# EUROPEAN PATENT SPECIFICATION

(11) **EP 2 313 489 B1**
(45) Date of publication and mention of the grant of the patent: **29.04.2015**
(21) Application number: 09780692.1
(22) Date of filing: 16.07.2009
(51) Int. Cl.: C12N 1/20

(54) **PRODUCTION METHOD**
HERSTELLUNGSVERFAHREN
PROCÉDÉ DE PRODUCTION

(30) Priority: 28.07.2008 EP 08161267; 28.08.2008 EP 08163201
(43) Date of publication of application: 27.04.2011
(73) Proprietor: B.R.A.I.N. Biotechnology Research and Information Network AG, 64673 Zwingenberg (DE)
(72) Inventor: MAMPEL, Jörg, 64625 Bensheim (DE); MEURER, Guido, 64342 Seeheim-Jugenheim (DE); ECK, Jürgen, 64625 Bensheim (DE)
(74) Representative: Vossius & Partner Patentanwälte Rechtsanwälte mbB
(86) International application number: PCT/EP2009/059132
(87) International publication number: WO 2010/012604

(56) References cited:
- WO-A-2008/006037
- ALTARAS N E ET AL: "Enhanced production of (R)-1,2-propanediol by metabolically engineered Escherichia coli" BIOTECHNOLOGY PROGRESS, AMERICAN INSTITUTE OF CHEMICAL ENGINEERS, US, vol. 16, no. 6, 1 November 2000 (2000-11-01), pages 940-946, XP002293971 ISSN: 8756-7938
- ALTARAS N E ET AL: "Metabolic engineering of a 1,2-Propanediol pathway in Escherichia coli" APPLIED AND ENVIRONMENTAL MICROBIOLOGY, AMERICAN SOCIETY FOR MICROBIOLOGY, US, vol. 65, no. 3, 1 March 1999 (1999-03-01), pages 1180-1185, XP002293970 ISSN: 0099-2240
- CONWAY T ET AL: "SIMILARITY OF ESCHERICHIA COLI PROPANEDIOL OXIDOREDUCTASE (FUCO PRODUCT) AND AN UNUSUAL ALCOHOL DEHYDROGENASE FROM ZYMOMONAS MOBILIS AND SACCHAROMYCES CEREVISIAE" JOURNAL OF BACTERIOLOGY, AMERICAN SOCIETY FOR MICROBIOLOGY, US, vol. 171, no. 7, 1 July 1989 (1989-07-01), pages 3754-3759, XP000982763 ISSN: 0021-9193

## Description

The present invention relates to a method for the production of 1,2-propanediol from glycerol in host cells as characterized in the appended claims. More specifically the present invention describes recombinant enzymatic activities which enable the synthesis of exclusively the 1,2-isomer of propanediol from pure and crude preparations of glycerol. The present invention also provides suitable combinations of overexpression and inactivation of key-activities for the production of 1,2-propanediol.

1,2-propanediol (propylene glycol; 1,2-PD) is a major bulk chemical that is widely used as a component of unsaturated polyester resins, pharmaceutical formulations and cosmetics, liquid detergents, coolants and anti-freeze or de-icing fluids. Since 1,2-PD is optically active, enantiomerically pure preparations of 1,2-PD might be of special interest for medical, agricultural or physiological applications.

1,2-propanediol is currently produced from petrochemicals by chemical synthesis that involves handling of large amounts of toxic compounds like epichlorhydrin or hydroperoxid. In conventional chemical synthesis, 1,2-PD is obtained by the hydration of propylene oxide, which is produced from propylene. The chemical synthesis yields racemic 1,2-PD and demands large amounts of water in order to prevent formation of polyglycol. Conventional chemical synthesis is dependent on fossil resources and leads to the production of large amounts of by-products; thus it appears problematic in terms of environmental and economical aspects.

It is known that 1,2 propanediol can be produced by microorganisms from sugars as substrates (Kluyver and Schellen, 1937) (Heath, E.C., 1962) (Altaras, N.E, 2001) (Tran Din, K, 198) (Cameron, D.C., 1986) (Cameron, D.C, 1998) (Park, Y.H., 2006; US 7,049,109 B2).

For example, Altaras and Cameron, 1999 describe the production of 1,2-PD from glucose by *E.coli strains* expressing glycerol dehydrogenase, methyglyoxal synthase or co-expressing both enzymes.

Altaras and Cameron, 2000 describe an alternative to the method of Altaras and Cameron, 1999 by using *E.coli* strains that were engineered to co-express methylglyoxal synthetase, glycerol dehydrogenase and either alcohol dehydrogenase or 1,2-propanediol oxidoreductase.

US 6087140 and US 6303352 describe the production of 1,2-PD from sugars except 6-deoxyhexoses by recombinant organisms.

In WO 2005/073364, US 2007/072279 a method is described by which microorganisms are generated and selected that show enhanced capabilities to produce 1,2-PD from unspecified carbon sources. More specifically, inactivation of a set of genes is taught to create strains harbouring single or multiple mutations that are the basis for a subsequent selection procedure by chemostat-fermentations. The focus of the application is on the inactivation of the genes encoding an aldA and gloA activity. All disclosed examples are given for *E. coli* MG1655 that has mutations in at least the following two genes: triosephosphat-isomerase (tpiA) and both subunits of pyruvate-formate lyase (pflAB). Furthermore, the examples specifically refer to glucose as carbon-source for fermentations.

There is therefore an unmet need in the art for improving the biotechnological processes for the production of 1,2-propanediol (1,2-PD). There is further an unmet need for improved microbial strains that can be used in such a process.

The present invention addresses this unmet need by providing solutions to the problems that had so far prevented significant improvements in this area.

To solve these problems, the present invention provides an improved biotechnological process for the production of 1,2 propanediol (1,2-PD) from a non-fermentable, inexpensive carbon substrate as characterized in the appended claims, whereby the carbon substrate is sustaining production of biomass and serves as a substrate for production of 1,2 propanediol (1,2-PD) at the same time. The present invention further provides improved microbial strains as characterized in the appended claims which are specifically adapted to the specific requirements of this procedure and are therefore specifically suited for use in the process according to the invention.

In particular, the present invention provides a host cell as characterized in the appended claims, particularly a microorganism or strain, which is engineered to produce high levels of 1,2 propanediol (1,2-PD) when grown on a non-fermentable carbon substrate, whereby the carbon substrate is sustaining production of biomass and serves as a substrate for production of 1,2 propanediol (1,2-PD) at the same time, wherein glycerol is the sole carbon source.

In one embodiment of the invention, a host cell, particularly a microorganism or strain, is provided which is engineered to produce high levels of 1,2 propanediol (1,2-PD) when grown on glycerol as the sole carbon source, wherein said glycerol has a degree of purity of at least 70%, particularly of at least 75%, particularly of at least 80%, particularly of at least 85%, particularly of at least 90%, particularly of at least 95%, particularly of at least 99% and up to 100%, with all integers falling within the above defined ranges also being comprised herewith.

In a specific embodiment, the glycerol has a degree of purity of between 80% and 90%, particularly of about 85%.

The invention provides a host cell, particularly a microorganism or strain, which is capable of producing high levels of 1,2 propanediol (1,2-PD) when grown on glycerol as the sole carbon source, wherein said host cell, particularly said microorganism or strain, is engineered to co-express a glycerol dehydrogenase (gldA) and a dihydroxyacetone kinase (dhaK) activity along with the propanediol oxidoreductase (fucO) activity, by co-introducing in said host cell together with the gene encoding a propanediol oxidoreductase (fucO) activity, genes encoding a glycerol dehydrogenase (gldA) and a dihydroxyacetone kinase (dhaK) activity such as to express said glycerol dehydrogenase (gldA) and dihydroxyacetone kinase (dhaK) activities along with the propanediol oxidoreductase (fucO) activity.

In one embodiment, the invention provides a host cell, particularly a microorganism or strain, which is capable of producing high levels of 1,2 propanediol (1,2-PD) when grown on glycerol as the sole carbon source, wherein said host cell, particularly said microorganism or strain, is engineered to co-express a glycerol dehydrogenase (gldA), a dihydroxyacetone kinase (dhaK) and a methylglyoxalsynthase (mgsA) activity along with the propanediol oxidoreductase (fucO) activity, particularly by co-introducing in said host cell together with the gene encoding a propanediol oxidoreductase (fucO) activity, genes encoding a glycerol dehydrogenase (gldA), a dihydroxyacetone kinase (dhaK) and a methylglyoxalsynthase (mgsA) activity such as to express said glycerol dehydrogenase (gldA), dihydroxyacetone kinase (dhaK) and methylglyoxalsynthase (mgsA) activities along with the propanediol oxidoreductase (fucO) activity.

In one embodiment, the invention provides a host cell, particularly a microorganism or strain, which is capable of producing high levels of 1,2 propanediol (1,2-PD) when grown on glycerol as the sole carbon source, wherein said host cell, particularly said microorganism or strain, is engineered to co-express a glycerol dehydratase activity along with the propanediol oxidoreductase (fucO) activity, the glycerol dehydrogenase activity and the dihydroxyacetone kinase activity particularly by co-introducing in said host cell together with the gene encoding a propanediol oxidoreductase (fucO) activity, a gene encoding a glycerol dehydrogenase activity, a gene encoding a dihydroxyacetone kinase activity and genes encoding a glycerol dehydratase activity such as to express said glycerol dehydratase activity along with the propanediol oxidoreductase (fucO) activity.

In one embodiment, the invention provides a host cell, particularly a microorganism or strain, which is capable of producing high levels of 1,2 propanediol (1,2-PD) when grown on glycerol as the sole carbon source, wherein said host cell, particularly said microorganism or strain, is engineered to co-express an aldo-keto-reductase activity along with the propanediol oxidoreductase (fucO) activity, the glycerol dehydrogenase activity and the dihydroxyacetone kinase activity particularly an aldo-keto-reductase activity, which is contributed by a gene, particularly a microbial gene, selected from the group consisting of dkgA, dkgB, yeaE and yghZ, particularly by co-introducing in said host cell together with the gene encoding a propanediol oxidoreductase (fucO) activity, a gene encoding a glycerol dehydrogenase activity, a gene encoding a dihydroxyacetone kinase activity and genes encoding an aldo-keto-reductase activity, particularly a microbial gene, selected from the group consisting of dkgA, dkgB, yeaE and yghZ such as to express said aldo-keto-reductase activity along with the propanediol oxidoreductase (fucO) activity the glycerol dehydrogenase activity and the dihydroxyacetone kinase activity.

In one embodiment, the invention provides a host cell, particularly a microorganism or strain, which is capable of producing high levels of 1,2 propanediol (1,2-PD) when grown on glycerol as the sole carbon source, wherein said host cell, particularly said microorganism or strain, has been engineered through recombinant DNA techniques.

In one embodiment, the invention provides a host cell, particularly a microorganism or strain according to the invention and as described herein before, which is capable of producing high levels of 1,2 propanediol (1,2-PD) when grown on glycerol as the sole carbon source, wherein said host cell, particularly a microorganism or strain, is defective in arabinose metabolism. In one embodiment, said host cell, particularly said microorganism or strain, is defective in arabinose metabolism due to a reduced or missing ribulose kinase activity.

In one embodiment, the invention provides a host cell, particularly a microorganism or strain according to the invention and as described herein before, which is capable of producing high levels of 1,2 propanediol (1,2-PD) when grown on glycerol as the sole carbon source, wherein said host cell, particularly said microorganism or strain, is defective in the metabolism of methylglyoxal. In one embodiment, said host cell, particularly said microorganism or strain, is defective in the metabolism of methylglyoxal due to a reduced or missing enzyme activity selected from the group consisting of glyoxylase system I, glyoxylase system II, lactate dehydrogenase A, glyoxylase system III, aldehyde dehydrogenase A activity, but especially due to a reduced or missing glyoxylase system I activity.

In one embodiment, the invention provides a host cell, particularly a microorganism or strain according to the invention and as described herein before, which is capable of producing high levels of 1,2 propanediol (1,2-PD) when grown on glycerol as the sole carbon source, wherein said host cell, particularly said microorganism or strain, is defective in the metabolism of dihydroxyacetonphosphate. In one embodiment, said host cell, particularly said microorganism or strain, is defective in the metabolism of dihydroxyacetonphosphate due to a reduced triosephosphate isomerase activity.

In one embodiment, the invention provides a host cell, particularly a microorganism or strain according to the invention and as described herein before, wherein said microorganism is *E. coli.*

In one embodiment, the invention provides a method for the preparation of 1,2-propanediol whereby a host cell, particularly a microorganism strain according to the invention is grown in an appropriate growth medium containing a simple carbon source, particularly a crude glycerol preparation, after which the 1,2-propanediol produced are recovered and, if necessary, purified.

In particular, the invention provides a method of producing 1,2-propanediol by growing a host cell, particularly a microorganism or strain according to the invention, on a non-fermentable carbon substrate, comprising:
i) culturing said host cell, particularly said microorganism or strain, according to the invention and as described herein before, which host cell overexpresses propanediol oxidoreductase (fucO) activity, a glycerol dehydrogenase activity and a dihydroxyacetone kinase activity in a medium containing a non-fermentable carbon substrate, whereby the carbon substrate is sustaining production of biomass and serves as a substrate for production of 1,2 propanediol (1,2-PD) at the same time, and the non-fermentable carbon source is metabolized by the host cell, particularly the microorganism or strain, according to the invention into 1,2-propanediol
ii) recovering the 1,2-propanediol produced according to step i); and, optionally,
iii) purifying the recovered 1,2-propanediol.

In one embodiment of the invention, said non-fermentable carbon substrate is a crude glycerol preparation, particularly a preparation containing glycerol with a purity of at least 70%, particularly of at least 75%, particularly of at least 80%, particularly of at least 85%, particularly of at least 90%, particularly of at least 95%, particularly of at least 99% and up to 100%.

In a specific embodiment, the glycerol has a degree of purity of between 80% and 90%, particularly of about 85%.

In one embodiment, the non-fermentable carbon substrate, particularly the crude glycerol preparation as described herein before, is selectively metabolized to 1,2-propanediol.

In one embodiment, the invention provides a method of producing 1,2-propanediol as described herein before, wherein a host cell, particularly a microorganism or strain, according to the invention and as described herein before is used in said process, which is engineered to overexpress propanediol oxidoreductase (fucO) a glycerol dehydrogenase activity and a dihydroxyacetone kinase activity.

In one embodiment, the invention provides a method of producing 1,2-propanediol as described herein before, wherein a host cell, particularly a microorganism or strain, according to the invention and as described herein before is used in said process which is engineered to co-express glycerol dehydrogenase (gldA), dihydroxyacetone kinase (dhaK) and methylglyoxalsynthase (mgsA) along with the propanediol oxidoreductase (fucO) activity.

In one embodiment of the invention, a host cell, particularly a microorganism or strain, according to the invention and as described herein before is used, wherein at least one enzyme activity involved in a non-productive pathway competing with 1,2-PD production has been deactivated.

In particular, microbial mutants, particularly mutants *of E. coli,* are used wherein one or more of the genes encoding glyoxylase systems I and II (gloA and gloB), lactate dehydrogenase A (ldhA), glyoxylase system III (indirectly by inactivation of the master regulator rpoS), and aldehyde dehydrogenase have been deactivated.

In another embodiment, a microbial mutant or strain, particularly an *E. coli* mutant, is used wherein the gene encoding a gloA activity has been partially or fully inactivated.

In another embodiment, a microbial mutant or strain inactivated in arabinose metabolism is used within the process according to the invention.

In one embodiment of the invention, an *E.coli* strain is used as the host organism, particularly an *E. coli* strain MG1655 and DH5alpha, respectively.

In one embodiment of the invention, at least one of the genes encoding an enzyme activity selected from the group consisting of glycerol dehydrogenase (gldA), dihydroxyacetone kinase (dhaK) and methylglyoxalsynthase (mgsA) and propanediol oxidoreductase (fucO) is under the control of an inducible promoter, particularly an arabinose inducible promoter, particularly a paraBAD promoter.

In one embodiment of the invention, a synthetic operon is used to provide a host cell, particularly a microorganism or strain as characterized in the appended claims, co-expressing at least one enzyme activity selected from the group consisting of glycerol dehydrogenase (gldA), dihydroxyacetone kinase (dhaK) and methylglyoxalsynthase (mgsA) activity along with the propanediol oxidoreductase (fucO) activity. In one embodiment of the invention, the genes encoding the above activities are under control of an inducible promoter, particularly an arabinose-inducible promoter, but especially a paraBAD promoter.

Described herein is a synthetic operon comprising the gene encoding propanediol oxidoreductase (fucO) and at least one additional gene encoding an enzyme protein selected from the group consisting of glycerol dehydrogenase, dihydroxyacetone kinase and methylglyoxalsynthase (mgsA), particularly a synthetic operon comprising the genes encoding propanediol oxidoreductase (fucO), glycerol dehydrogenase, dihydroxyacetone kinase and methylglyoxalsynthase (mgsA).

Further described herein is that the synthetic operon is under the control of an inducible promoter, particularly an arabinose-inducible promoter.

In one embodiment of the invention, the genes encoding the succession of genes transcribed upon induction from said operon is as follows: *mgsA, gldA, dhaK, fucO.*

Further described herein are polynucleotide molecules or constructs, particularly plasmids and vector molecules, comprising the synthetic operon and host cells, particularly microbial host cells comprising said polynucleotide molecules.

### BRIEF DESCRIPTION OF THE DRAWING AND SEQUENCE LISTING

- Figure 1: illustrates inhibition of growth of wild type (black bars) and mutant strains (deltagloA-mutant, grey-bars; deltagloB-mutant, hatched bars) of *E. coli* by different amounts of methylglyoxal added to the culture broth

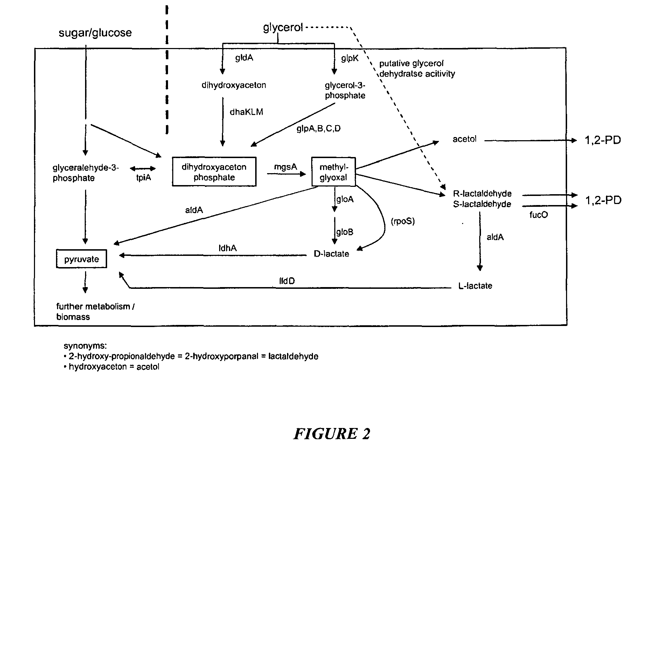

- Figure 2: is a schematic drawing of pathways generating 1,2-PD when sugars or glycerol are carbon substrates

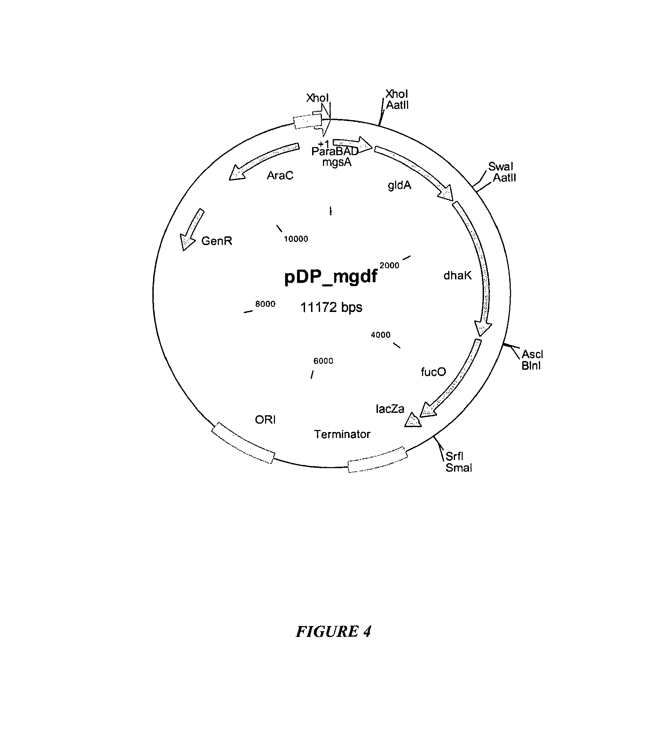

- Figures 3 & 4: illustrate maps of plasmids described within the invention

### DEFINITIONS

The term "polynucleotide" is understood herein to refer to polymeric molecule of high molecular weight which can be single-stranded or double-stranded, composed of monomers (nucleotides) containing a sugar, phosphate and a base which is either a purine or pyrimidine. The term "polynucleotide " thus primarily refers to a polymer of DNA or RNA which can be single- or double-stranded, optionally containing synthetic, non-natural or altered nucleotide bases capable of incorporation into DNA or RNA polymers. Unless otherwise indicated, a particular nucleic acid sequence also implicitly encompasses conservatively modified variants thereof (e.g. degenerate codon substitutions) and complementary sequences and as well as the sequence explicitly indicated. Specifically, degenerate codon substitutions may be achieved by generating sequences in which the third position of one or more selected (or all) codons is substituted with mixed-base and/or deoxyinosine residues (Batzer, et al. (1991); Ohtsuka, et al., (1985); and Rossolini, et al. (1994)). The term polynucleotide is used interchangeably with nucleic acid, nucleotide sequence and may include genes, cDNAs, and mRNAs encoded by a gene, etc.

The term "construct" refers to a plasmid, virus, autonomously replicating sequence, phage or nucleotide sequence, linear or circular, of a single- or double-stranded DNA or RNA, derived from any source, in which a number of nucleotide sequences have been joined or recombined into a unique construction which is capable of introducing a promoter fragment and DNA sequence for a selected gene product encoding an enzyme activity according to the invention along with appropriate 3' untranslated sequence into a cell.

The term "transformation" or "transfection" refers to the acquisition of new genes in a cell after the incorporation of nucleic acid.

The term "expression" refers to the transcription and translation to gene product from a gene coding for the sequence of the gene product. In the expression, a DNA chain coding for the sequence of gene product is first transcribed to a complimentary RNA which is often a messenger RNA and, then, the thus transcribed messenger RNA is translated into the above-mentioned gene product if the gene product is a protein.

The term "plasmid" or "vector" or "cosmid" as used herein refers to an extra chromosomal element often carrying genes which are not part of the central metabolism of the cell, and usually in the form of circular double-stranded DNA molecules.

The term "regulator" used in the present specification refers to a base sequence having a functional promoter and any related transcriptional element (e.g., enhancer, CCAAT box, TATA box, SPI moiety and the like).

The term "operably linked" used in the present specification means that various regulatory elements such as a promoter, an enhancer and the like, that control the gene expression, and a gene of interest are connected in an operable state in a host cell such as to enable expression of said gene of interest. It is a well known matter to those of ordinary skill in the art that the type and kind of regulator can vary depending on the host.

The term 'deletion' denotes the suppression of the activity of a gene, which in general consists of a suppression of activity that can be an inactivation, an inhibition, or it can be the deletion of at least a part of the gene concerned (for example deletion of all or a part of the promoter region necessary for its expression) so that it is not expressed or non-functional or so that the expression product loses its function (for example deletion in a coding part of the gene concerned). Preferentially, the deletion of a gene is essentially the suppression of that gene, which gene can be replaced by a selection marker gene that facilitates the identification, isolation and purification of the strains according to the invention. For example, a gene may be inactivated by homologous recombination mediated by the recA-protein of e.g. E. coli (Cunningham, et al. (1980)).

Briefly, an inactivation protocol can be as follows: a linear fragment of DNA is introduced into the cell. This fragment is obtained *in vitro*, and comprises two regions flanking the gene, and a gene encoding a selectable gene product (generally an antibiotic-resistance gene) located between the two flanking regions. This fragment thus presents an inactivated gene. The cells that have undergone a recombination event and integrated the synthetic fragment are selected by plating on a selective growth medium. Cells that have undergone a double recombination event, in which the native gene has been replaced by the inactivated gene, are selected.

The term "carbon substrate" means any carbon source capable of being metabolized by a microorganism wherein the substrate contains at least one carbon atom.

The term "non-fermentable carbon substrate" as used in the present invention refers to carbon substrates that do not sustain redox-processes of a given organism to generate biomass in absence of exogenous electron acceptors.

1,2-propanediol (propylene glycol; 1,2-PD) is a major bulk chemical that is widely used as a component of unsaturated polyester resins, pharmaceutical formulations and cosmetics, liquid detergents, coolants and anti-freeze or de-icing fluids. Since 1,2 propanediol (1,2-PD) is optically active, enantiomerically pure preparations of 1,2 propanediol (1,2-PD) might be of special interest for medical, agricultural or physiological applications.

1,2 propanediol (1,2-PD) can be produced by microorganisms from sugars as substrates and sole carbon source. In recent years, alternative substrates such as glycerol have attracted considerable attention for use as fermentation substrate instead of e.g. sugar carbon sources. The interest in glycerol essentially is the result of significantly increased biodiesel or bio-ethanol production. Both processes generate glycerol as major by-product that makes up e.g. 10 % (w/w) of the biodiesel produced.

The present invention now provides an improved biotechnological process for the production of 1,2 propanediol (1,2-PD) from a non-fermentable, inexpensive carbon substrate, particularly a crude glycerol preparation, whereby the carbon substrate is sustaining production of biomass and serves as a substrate for production of 1,2 propanediol (1,2-PD) at the same time. The present invention further provides improved microbial strains which are specifically adapted to the specific requirements of this procedure and are therefore specifically suited for use in the process according to the invention.

In a preferred embodiment, the present invention provides for bioconverting glycerol or crude glycerol preparations as a non-fermentable carbon source directly to 1,2-propanediol using a host cell, particularly a microorganism or strain, that has been engineered to contain genes that are involved in the production pathway of 1,2 propanediol (1,2-PD) from glycerol. In particular, the host cell, particularly the microorganism or strain, according to the invention has been engineered by recombinant DNA techniques to produce a recombinant host cell, particularly a recombinant microorganism or strain, comprising genes involved in the metabolism of dihydroxyaceton phosphate and methylglyoxal, two key precursor compounds in the production pathway to 1,2 propanediol (1,2-PD). In particular, a host cell, particularly a microorganism or strain, is provided harbouring genes encoding enzymes exhibiting a glycerol-dehydrogenase activity, a dihydroxyaceton-kinase activity, a methylgyoxal-synthase activity and a propanedioloxidoreductase activity, which enzymes are able to convert glycerol to 1,2 propanediol (1,2-PD) with high selectivity.

In a preferred embodiment, an engineered *E.coli* strain, particularly a recombinant *E. coli* strain is used within the scope of the present invention.

It was surprisingly found within the present invention that common crude-glycerol (85% purity) from biodiesel production can be used as substrate for growth of a broad variety of organisms of different origin under oxic and anoxic conditions. It could be demonstrated that most of the organisms tested were not impaired by crude glycerol compared to pure glycerol with regard to biomass production, indicating that crude glycerol can be utilised and is in general not toxic to microorganisms. Biomass production was not affected by the impurities found in crude-glycerol preparations. Crude glycerol from biodiesel-production or alternative sources can, therefore, be equally well utilised as carbon-source by microorganisms and thus can be used without further processing as a general renewable carbon source in fermentation processes for biomass production.

Crude glycerol preparations, particularly crude glycerol preparations from biodiesel or bioethanol production may therefore be used in the process according to the present invention for producing 1,2 propanediol (1,2-PD).

A first key precursor compound in the production pathway to 1,2 propanediol (1,2-PD) is dihydroxyaceton phosphate (DHAP). DHAP is converted to methylglyoxal, a 2^{nd} essential precursor compound, through the activity of a methylglyoxal synthase (mgsA). The methylglyoxal becomes finally converted into S-lactaldehyde. A so far unidentified glycerol-dehydratase activity may convert glycerol into R- or S-lactaldehyde, which is further metabolised to R- or S-1,2-PD, respectively. Whereas an endogenous reductive activity of the host cell is proposed to produce R-1,2-PD from the R-lactaldehyde, the S-lactaldehyde appears to be the substrate for the propanediol oxidoreductase (fucO), which may convert S-lactaldehyde into S-1,2-PD (Altaras, N.E., 1999; Applield and Environmental Microbiology (65), 1180-1185).

It was therefore hypothesized that by introducing propanediol oxidoreductase (fucO) into a host organism the flexibility of the 1,2 propanediol (1,2-PD) producing network may be expanded by accepting the S-entantiomer of lactaldehyde for conversion to 1,2 propanediol (1,2-PD). Furthermore, it was concluded that propanediol oxidoreductase (fucO) activity might be necessary for production of 1,2 propanediol (1,2-PD) from glycerol independent of the methylglyoxal pathway.

Accordingly, a wild-type strain that does not produce detectable amounts of 1,2 propanediol (1,2-PD) from glycerol, irrespective of the conditions for cultivation, was supplemented with a polynucleotide comprising a nucleotide sequence encoding a propanediol oxidoreductase (fucO) activity.

In one embodiment of the invention, the gene encoding a propanediol oxidoreductase (fucO) activity was cloned into a host organism which does not produce detectable amounts of 1,2 propanediol (1,2-PD) from glycerol and over-expressed in said host in minimal medium containing glycerol under oxic and semi anoxic conditions. Overexpression of propanediol oxidoreductase (fucO) activity resulted in production of 1,2 propanediol (1,2-PD).

A further key precursor compound in the production pathway to 1,2 propanediol (1,2-PD) is dihydroxyacetone phosphate (DHAP). According to the invention, an alternative pathway to yield dihydroxyacetone phosphate as precursor for 1,2 propanediol (1,2-PD)-synthesis is engineered into a microbial strain, particularly into an *E. coli* strain, which pathway produces the essential precursor DHAP independent of the endogenous regulatory network acting on glycerolphosphate kinase (glpK).

In particular, a DNA molecule comprising a nucleotide sequence encoding a glycerol dehydrogenase (gldA) and dihydroxyacetone kinase (dhaK) activity is introduced in a host organism, particularly a *E. coli* host. The gene encoding the glycerol dehydrogenase (gldA) may be isolated from an *E .coli* strain, particularly an *E. coli* K12, and cloned into a suitable plasmid.

In one embodiment, the gene encoding the glycerol dehydrogenase (gldA) may be cloned into a suitable plasmid along with a gene encoding dihydroxyacetone kinase (dhaK) activity. The gene encoding the dihydroxyacetone kinase (dhaK) activity is isolated from a *Citrobacter* strain, particularly a *Citrobacter freundii* strain.

In another embodiment of the invention, the glycerol dehydrogenase (gldA) gene is cloned into a suitable plasmid independent of and separate from the dihydroxyacetone kinase (dhaK).

In one embodiment of the invention, the introduced coding sequences encoding a glycerol dehydrogenase (gldA) and/or a dihydroxyacetone kinase (dhaK) activity are under control of an inducible promoter, particularly an arabinose inducible promoter (paraBAD).

The genes of this alternative pathway to yield dihydroxyacetone phosphate encoding the glycerol dehydrogenase (gldA) and the dihydroxyacetone kinase (dhaK) activity, respectively, may be introduced into a wild-type host organism together with a polynucleotide comprising the nucleotide sequence encoding the propanediol oxidoreductase (fucO) activity, either separately as individual expression cassettes, wherein the coding sequence is under control of its own promoter and termination signal, which expression cassettes may either be located on different plasmids or on a single plasmid, or in form of a synthetic operon comprising two or more of said genes under the control of common promoter and termination sequences.

In one embodiment of the invention, the genes encoding the glycerol dehydrogenase (gldA) and dihydroxyacetone kinase (dhaK) activity are cloned to a single plasmid which already comprises a gene encoding the propanediol oxidoreductase (fucO) activity to create a plasmid comprising a gene encoding a glycerol dehydrogenase (gldA) along with the propanediol oxidoreductase (fucO) activity, or a plasmid comprising a gene encoding a dihydroxyacetone kinase (dhaK) along with the propanediol oxidoreductase (fucO) activity.

In one embodiment, a plasmid is created, which comprises a gene encoding a glycerol dehydrogenase (gldA) and a dihydroxyacetone kinase (dhaK) along with the propanediol oxidoreductase (fucO) activity.

The various gene sequences encoding the different enzyme activities may be arranged on the plasmid such as to create a synthetic operon, wherein two or more genes are arranged under the control of common regulatory sequences including promoter and polyadenylation sequences. In one embodiment, the synthetic operon is under control of an inducible promoter, particularly an arabinose-inducible promoter.

DHAP is the initial intermediate in the pathway generating 1,2 propanediol (1,2-PD). Triosephosphateisomerase (tpi) of the glycolytic pathway competes with methylglyoxal synthase for DHAP. In order to drive 1,2 propanediol (1,2-PD) production, mgsA encoding methylglyoxal synthase may be incorporated in the synthetic operon in order to shift the balance towards 1,2 propanediol (1,2-PD) production.

Further described is an extended synthetic operon comprising in addition to the genes involved in the dihydroxyaceton phosphate pathway an additional gene involved the production of methylglyoxal, particularly a methylgyoxal-synthase gene, particularly a methylgyoxal-synthase gene *of E. coli.*

The resulting plasmid(s) is(are) then introduced in a host cell, particularly a microbial host cell or strain, which is unable of producing detectable amounts of 1,2 propanediol (1,2-PD) from glycerol, irrespective of the conditions for cultivation, particularly in an *E. coli* strain.

In one embodiment of the invention, the host organism has no active arabinose metabolism or has previously been inactivated in arabinose metabolism by deleting or inactivating at least one of the essential genes involved in the arabinose metabolism such as, for example, the gene encoding ribulose-kinase activity (araB). The corresponding strains are cultivated in minimal medium containing glycerol under oxic and semi anoxic conditions, and the 1,2 propanediol (1,2-PD) is isolated from the supernatants and analysed.

Overexpression of the propanediol oxidoreductase gene (fucO) results in production of 1,2 propanediol (1,2-PD) from crude glycerol preparations. The amounts of 1,2 propanediol (1,2-PD) can be increased by co-expression of a dihydroxyacetone kinase (dhaK) and a glycerol dehydrogenase gene (gldA) together with the propanediol oxidoreductase gene (fucO). Further improvements may be achieved by co-expression of a dihydroxyacetone kinase (dhaK) and a glycerol dehydrogenase gene (gldA) and a methylgyoxal-synthase gene (mgsA) together with the propanediol oxidoreductase gene (fucO) and by the use of host cells, particularly microbial host cells or strains, which are defective in at least one of the non-productive pathways competing for key precursor compounds in the 1.2 - propanediol production pathway.

The arrangement of the genes involved in catalysis in the described manner as 5'-mgsA, gldA, dahK, fucO-3' is preferred, however the invention is not restricted to this specified arrangement. Any order of the described genes might be suitable for 1,2 propanediol (1,2-PD) production.

The pathway was demonstrated to be specific for the production of the 1,2 propanediol (1,2-PD) isomer of propanediol, since no 1,3-propanediol was detected.

Methods of obtaining desired genes from a bacterial genome are common and well known in the art of molecular biology. For example, if the sequence of the gene is known, suitable genomic libraries may be created by restriction endonuclease digestion and may be screened with probes complementary to the desired gene sequence. Once the sequence is isolated, the DNA may be amplified using standard primer directed amplification methods such as polymerase chain reaction (PCR) (U.S. Pat. No. 4,683,202) to obtain amounts of DNA suitable for transformation using appropriate vectors. Alternatively, cosmid libraries may be created where large segments of genomic DNA (35-45 kb) may be packaged into vectors and used to transform appropriate hosts. Cosmid vectors are unique in being able to accommodate large quantities of DNA. Generally, cosmid vectors have at least one copy of the cos DNA sequence which is needed for packaging and subsequent circularization of the foreign DNA. In addition to the cos sequence these vectors will also contain an origin of replication such as ColE1 and drug resistance markers such as a gene resistant to ampicillin or neomycin. Methods of using cosmid vectors for the transformation of suitable bacterial hosts are well described in Sambrook et al., (1989). Typically to clone cosmids, foreign DNA is isolated and ligated, using the appropriate restriction endonucleases, adjacent to the cos region of the cosmid vector. Cosmid vectors containing the linearized foreign DNA are then reacted with a DNA packaging vehicle such as bacteriophage 1. During the packaging process the cos sites are cleaved and the foreign DNA is packaged into the head portion of the bacterial viral particle. These particles may then be used to transfect suitable host cells such as *E. coli.* Once injected into the cell, the foreign DNA circularizes under the influence of the cos sticky ends. In this manner large segments of foreign DNA can be introduced and expressed in recombinant host cells.

Once a gene has been isolated and its sequences put into the public domain, the references given, for example, on GenBank for these known genes can be used by those skilled in the art to determine the equivalent genes in other organisms, bacterial strains, yeasts, fungi, mammals and plants, etc. This routine work is advantageously performed using consensus sequences that can be determined using sequence alignments with genes from other micro-organisms, and by designing degenerate probes by means of which the corresponding gene can be cloned in another organism. These routine techniques of molecular biology are well known to the art and are described, for example, in Sambrook et al. (1989).

Described herein are a variety of vectors and transformation and expression cassettes suitable for the cloning, transformation and expression of the enzymatic activities according to the invention.

Said vector may be, for example, a phage, plasmid, viral or retroviral vector. Retroviral vectors may be replication competent or replication defective. In the latter case, viral propagation generally will occur only in complementing host/cells.

The polynucleotides or genes may be joined to a vector containing selectable markers for propagation in a host. Generally, a plasmid vector is introduced in a precipitate such as a calcium phosphate precipitate or rubidium chloride precipitate, or in a complex with a charged lipid or in carbon-based clusters, such as fullerens. Should the vector be a virus, it may be packaged in vitro using an appropriate packaging cell line prior to application to host cells.

Further described herein is that in the vector the polynucleotide is operatively linked to expression control sequences allowing expression in prokaryotic or eukaryotic cells or isolated fractions thereof.

Expression of said polynucleotide comprises transcription of the polynucleotide, preferably into a translatable mRNA. Regulatory elements ensuring expression in eukaryotic cells such as a bacterial or fungal cells, an insect cells, an animal cells, mammalian cells or a human cells, but particularly bacterial or fungal cells, are well known to those skilled in the art. They usually comprise regulatory sequences ensuring initiation of transcription and optionally poly-A signals ensuring termination of transcription and stabilization of the transcript. Additional regulatory elements may include transcriptional as well as translational enhancers. Possible regulatory elements permitting expression in prokaryotic host cells comprise, e.g., the *lac, trp* or *tac* promoter in *E. coli,* and examples for regulatory elements permitting expression in eukaryotic host cells are the *AOX1* or *GAL1* promoter in yeast. Beside elements which are responsible for the initiation of transcription such regulatory elements may also comprise transcription termination signals, downstream of the polynucleotide.

In this context, suitable expression vectors are known in the art such as Okayama-Berg cDNA expression vector pcDV1 (Pharmacia), pCDM8, pRc/CMV, pcDNA1, pcDNA3 (In-vitrogene), pSPORT1 (GIBCO BRL), pSE380 (In-vitrogene), or any pBR322 or pUC18-derived plasmids. Preferably, said vector is an expression vector and/or a gene transfer or targeting vector. Expression vectors derived from viruses such as retroviruses, vaccinia virus, adeno-associated virus, herpes viruses, or bovine papilloma virus, may be used for delivery of the polynucleotides or vector into targeted cell population. Methods which are well known to those skilled in the art can be used to construct recombinant viral vectors; see, for example, the techniques described in Sambrook, (1989) and Ausubel (1994). Alternatively, the polynucleotides and vectors can be reconstituted into liposomes for delivery to target cells.

Suitable host cells for the recombinant production of 1,2-propanediol may be either prokaryotic or eukaryotic and will be limited only by the host cell ability to express active enzymes.

Said host cell may be a prokaryotic or eukaryotic cell. The polynucleotide or vector which is present in the host cell may either be integrated into the genome of the host cell or it may be maintained extrachromosomally. Also described herein are recombinant DNA molecules that can be used for "gene targeting" and/or "gene replacement", for restoring a mutant gene or for creating a mutant gene via homologous recombination; see for example Mouellic, (1990); Joyner, Gene Targeting, A Practical Approach, Oxford University Press.

The host cell can be any prokaryotic or eukaryotic cell, such as a bacterial or fungal cell, an insect cell, an animal cell, a mammalian cell or a human cell, but particularly a bacterial or fungal cell. Preferred hosts will be those typically useful for production of glycerol or 1,2-propanediol. Preferred fungal cells are, for example, those of the genus Aspergillus, Saccharomyces, Schizosaccharomyces, Zygosaccharomyces, Pichia, Kluyveromyces, Candida, Hansenula, Debaryomyces, Mucor and Torulopsis, in particular those of the species *S. cerevisiae.* The term "prokaryotic" is meant to include all bacteria and archaea which can be transformed or transfected with a polynucleotide for the expression of an enzyme activity according to the present invention. Prokaryotic hosts may include gram negative as well as gram positive bacteria such as, for example Citrobacter, Enterobacter, Clostridium, Klebsiella, Aerobacter, Lactobacillus, Methylobacter, Escherichia, Salmonella, Bacillus, Streptomyces and Pseudomonas. Most preferred in the present invention are *E. coli, S. typhimurium, Serratia marcescens* and *Bacillus subtilis, Klebsiella* species and *Saccharomyces* species, but particularly *E. coli* species.

Specific examples thereof include *Escherichia coli* MG1655 (ATCC 700926; Bachmann, B., pp. 2460-2488 in Neidhardt et al.1996), *Escherichia coli* XL1-Blue MRF' [manufactured by Stratagene, Strategies, 5, 81 (1992)], Escherichia coli C600 [Genetics, 39, 440 (1954)], *Escherichia coli* Y1088 [Science, 222, 778 (1983)], *Escherichia coli* Y1090 [Science, 222, 778 (1983)], *Escherichia coli* NM522 [J. Mol. Biol., 166, 1 (1983)], *Escherichia coli* K802 [J. Mol. Biol., 16, 118 (1966)], *Escherichia coli* JM109 [Gene, 38, 275 (1985)], *Escherichia coli* DH5α [J. Mol. Biol., 166, 557 (1983)], and the like.

Further improvements may be achieved in 1,2-PD production by the use of suitable microbial mutants, wherein some or all enzyme activities involved in non productive pathways have been reduced or eliminated. For example, the enzymatic activities encoded by mgsA and tpi compete for DHAP. Methylglyoxal-synthase activity (MgsA) was shown to be inactivated by diphosphate (Hopper, D.J. 1972). In order to improve conversion of DHAP into methylglyoxal, a phosphate-insensitive mutant of MgsA can be identified by screening variant libraries obtained by any method generating variation within coding-sequences of mgsA, e.g. error-prone PCR. Microbial strains, particularly *E. coli* clones, previously inactivated in triosephosphate isomerase (tpiA) and endogenous mgsA, are transformed with plasmid libraries of mgsA-variants and grown on non-selective solid-media. By replica-plating of the initial transformants (plate A) on agar-plates containing high concentrations of glycerol or DHAP (plate B) or high-concentrations of diphosphate and glycerol or DHAP (plate C), clones that can grow on plate A and B, but not on plate C will be selected. These clones encode mgsA-variants with significant activity that produce toxic levels of methylglyoxal in presence of high concentrations of phosphate.

Triosephosphate isomerase mutants may be generated as described for methylglyoxalsynthase mutants. Tpi-mutants that are significantly impaired concerning growth kinetics are identified comparing growth kinetics on complex medium (e.g. Luria Broth, LB), glucose and glycerol. The mutant of interest shows slower or no growth compared to the unmodified strain with glycerol as sole source for carbon and energy, whereas growth kinetics with LB or glucose as carbon-source is unaffected.

Two other major routes for the detoxification of MG exist that are productive in terms of 1,2 PD biosynthesis. They are catalysed by so called MG-reductases as initial step. Several enzymes are proposed to encode this activity that should be strengthened by the inactivation of the competing, non-productive pathways.

In one embodiment of the invention, microbial mutants, particularly mutants of *E. coli,* are constructed wherein one or more of the genes encoding glyoxylase systems I and II (gloA and gloB), lactate dehydrogenase A (ldhA), glyoxylase system III (indirectly by inactivation of the master regulator rpoS), and aldehyde dehydrogenase A (aldA) have been inactivated such as to significantly reduce or completely inhibit expression of functional enzyme activities, through, for example, single gene knock-outs. This way, a microbial mutant can be obtained which has one or more of the mentioned genes inactivated, particularly a mutant wherein 2, particularly 3, particularly 4, particularly 5 of the genes selected from the group consisting of the genes encoding glyoxylase system I (gloA), glyoxylase systems II (gloB), lactate dehydrogenase A (ldhA), glyoxylase system III (indirectly by inactivation of the master regulator rpoS), and aldehyde dehydrogenase A (aldA) are inactivated.

In one embodiment of the invention, a microbial mutant, particularly an *E. coli* mutant, is provided wherein the gene encoding a gloA activity has been partially or fully inactivated.

In one embodiment of the present invention, a host cell, particularly a microorganism or strain, particularly a prokaryotic microorganism, e.g. *E. coli,* is inactivated in its ability to metabolise methylglyoxal (MG) into D- and/or L-lactate (MG-to-lactate metabolism). This is achieved by e.g. inactivation of glyoxylase A, preferably in combination with inactivation of one or more of the genes encoding glyoxylase B, the alternative sigma-factor rpoS and aldehyde-reductase A. In a preferred embodiment, a strain is deficient of all the above listed activities and/or genes.

In another embodiment, a strain inactivated in arabinose metabolism and in MG-to lactate metabolism, e.g. by inactivation of glyoxylase A, is transformed with a polynucleotide comprising the genes encoding an an enzyme activity selected from the group consisting of methylglyoxalsynthase (mgsA), glycerol dehydrogenase (gldA), dihydroxyacetone kinase (dhaK) and propanediol oxidoreductase (fucO) activity, particularly with plasmid pDP_mgdf, whereas the arrangement of the genes in the synthetic operon is not limited to that shown in plasmid pDP_mgdf, but can be in any order. The invention also refers to a strain not disabled in arabinose metabolism, e.g. wild type *E. coli.*

In another embodiment, a strain preferably but not necessarily inactivated in MG-to-lactate metabolism is transformed with plasmid-encoded genes that confer aldo-keto-reductase activity. The activity encoding genes are taken from a group *of E. coli* genes comprising dkgA, dkgB, yeaE and yghZ.

In a preferred embodiment, a strain expressing glycerol dehydrogenase (gldA), dihydroxyacetone kinase, propanediol oxidoreductase (fucO) and methylglyoxal synthase (e.g. by plasmid pDP_mgdf) is transformed with a plasmid encoding aldo-keto-reductase activity (e.g. DkgA of *E. coli*) and cultivated in a medium containing crude glycerol as carbon source.

In a specially preferred embodiment, a strain inactivated in MG-to-lactate metabolism and expressing glycerol dehydrogenase (gldA), dihydroxyacetone kinase, propanediol oxidoreductase (fucO) and methylglyoxal synthase (e.g. encoded on plasmid pDP_mgdf) expresses aldo-keto-reductase activity (e.g. dkgA of *E. coli* encoded on plasmid pCR2.1) and cultivated in a medium containing crude glycerol as carbon source.

The invention also refers to a strain not disabled in arabinose metabolism, and / or to strains expressing relevant enzyme activities cited within this invention from the chromosome of the microorganism.

A polynucleotide coding for an enzyme activity can be used to transform or transfect the host cell using any of the techniques commonly known to those of ordinary skill in the art.

The technique preferentially used to introduce these genes into the strain is electroporation, which is well known to those skilled in the art. Briefly, an electroporation protocol can be as follows: the heterologous genes of interest are cloned in an expression vector between a promoter and a terminator. This vector also possesses an antibiotic resistance gene to select cells that contain it and a functional replication origin in the host strain so it can be maintained. The protocol requires the preparation of electrocompetent host cells, which are then converted by electroporation by the vector. According to the invention, the genes introduced by electroporation are preferentially the genes according to the invention encoding an enzyme activity selected from the group consisting of glycerol dehydrogenase (gldA), dihydroxyacetone kinase (dhaK), methylglyoxalsynthase (mgsA) and propanediol oxidoreductase (fucO) activity.

Methods for preparing fused, operably linked genes and expressing them in bacteria or animal cells are well-known in the art (Sambrook, supra). The genetic constructs and methods described therein can be utilized for expression of polypeptides in, e.g., prokaryotic hosts. In general, expression vectors containing promoter sequences which facilitate the efficient transcription of the inserted polynucleotide are used in connection with the host. The expression vector typically contains an origin of replication, a promoter, and a terminator, as well as specific genes, which are capable of providing phenotypic selection of the transformed cells. The transformed prokaryotic hosts can be grown in fermentors and cultured according to techniques known in the art to achieve optimal cell growth.

Typically, cells are grown at 30° C in appropriate media. Preferred growth media in the present invention are defined or synthetic, e.g. minimal medium M9 containing glycerol as carbon source. Common commercially prepared media such as Luria Bertani (LB) broth, Sabouraud Dextrose (SD) broth or Yeast Malt Extract (YM) broth may also be used and the appropriate medium for growth of the particular microorganism will be known by a person skilled in the art of microbiology or fermentation science. The use of agents known to modulate catabolite repression directly or indirectly, e.g., cyclic adenosine 2':3'-monophosphate or cyclic adenosine 2':5'-monophosphate, may also be incorporated into the reaction media. Similarly, the use of agents known to modulate enzymatic activities (e.g., sulphites, bisulphites and alkalis) that lead to enhancement of 1,2-PD production may be used in conjunction with or as an alternative to genetic manipulations.

Suitable pH ranges for the fermentation are between pH 5.0 to pH 9.0, where pH 6.0 to pH 8.0 is preferred as range for the initial condition. Reactions may be performed under aerobic, microaerobic or anaerobic conditions where aerobic or microaerobic conditions are preferred.

*Batch and Continuous Fermentations:* The present process uses a batch method of fermentation. A classical batch fermentation is a closed system where the composition of the media is set at the beginning of the fermentation and not subject to artificial alterations during the fermentation. Thus, at the beginning of the fermentation the media is inoculated with the desired organism or organisms and fermentation is permitted to occur adding nothing to the system. Typically, however, a batch fermentation is "batch" with respect to the addition of the carbon source and attempts are often made at controlling factors such as pH and oxygen concentration. The metabolite and biomass compositions of the batch system change constantly up to the time the fermentation is stopped. Within batch cultures cells moderate through a static lag phase to a high growth log phase and finally to a stationary phase where growth rate is diminished or halted. If untreated, cells in the stationary phase will eventually die. Cells in log phase generally are responsible for the bulk of production of end product or intermediate. A variation on the standard batch system is the Fed-Batch fermentation system which is also suitable in the present invention. In this variation of a typical batch system, the substrate is added in increments as the fermentation progresses. Fed-Batch systems are useful when catabolite repression is apt to inhibit the metabolism of the cells and where it is desirable to have limited amounts of substrate in the media. Measurement of the actual substrate concentration in Fed-Batch systems is difficult and is therefore estimated on the basis of the changes of measurable factors such as pH, dissolved oxygen and the partial pressure of waste gases such as CO2. Batch and Fed-Batch fermentations are common and well known in the art and examples may be found in Brock, supra. It is also contemplated that the method would be adaptable to continuous fermentation methods. Continuous fermentation is an open system where a defined fermentation media is added continuously to a bioreactor and an equal amount of conditioned media is removed simultaneously for processing. Continuous fermentation generally maintains the cultures at a constant high density where cells are primarily in log phase growth. Continuous fermentation allows for the modulation of one factor or any number of factors that affect cell growth or end product concentration. For example, one method will maintain a limiting nutrient such as the carbon source or nitrogen level at a fixed rate and allow all other parameters to moderate. In other systems a number of factors affecting growth can be altered continuously while the cell concentration, measured by media turbidity, is kept constant. Continuous systems strive to maintain steady state growth conditions and thus the cell loss due to media being drawn off must be balanced against the cell growth rate in the fermentation. Methods of modulating nutrients and growth factors for continuous fermentation processes as well as techniques for maximizing the rate of product formation are well known in the art of industrial microbiology and a variety of methods are detailed by Brock, supra. The present invention may be practiced using either batch, fed-batch or continuous processes and that any known mode of fermentation would be suitable. Additionally, it is contemplated that cells may be immobilized on a substrate as whole cell catalysts and subjected to fermentation conditions for 1,2-propanediol production.

The 1,2 propanediol product can then be isolated from the grown medium or cellular lysates. The isolation and purification of the microbially or otherwise produced propanediols may be by any conventional means. Methods for the purification of propanediols from fermentation or cultivation media are known in the art. For example, propanediols can be obtained from cell media by subjecting the reaction mixture to extraction with an organic solvent, distillation and column chromatography (U.S. Pat. No. 5,356,812). A particularly good organic solvent for this process is cyclohexane (U.S. Pat. No. 5,008,473).

For industrial applications, purification of 1,2-propanediol from large volumes of fermentor broth requires non-laboratory scale methods. Difficulties to be overcome include removal of cell matter form the broth (clarification), concentration of 1,2-propanediol either by extraction or water removal and separation of residual impurities from the partially purified monomer. Broth clarification will typically proceed either by filtration, centrifugation or crossflow microfiltration. Suitable filters are manufactured for example by Millipore (Millipore Corporation, 80 Ashby Road, Bedford, Mass.) or Filmtec (Dow Chemical Co.). Centrifugation effectively removes the bulk of the cells, but, depending upon the nature of the broth, does not always achieve complete cell removal. Crossflow microfiltration yields extremely clear filtrate. The concentrate is a slurry rather than a high-solids cake. The skilled person will be able to adapt the clarification method most appropriate for the fermentation apparatus and conditions being employed. Water reduction of the clarified broth is complicated by the high solubility of 1,2-propanediol in water. Extraction of 1,2-propanediol from the clarified broth may be accomplished by a variety of methods, including evaporation/distillation, membrane technology, extraction by organic solvent and adsorption. Rotary evaporators may be used to initially reduce water volume in the clarified broth. This method has enjoyed good success in Applicants' hands. Precipitation of extraneous proteins and salts do not appear to affect 1,2-propanediol recovery Membrane technology may be used either separately or in conjunction with evaporation. Suitable membranes will either (i) allow passage of 1,2-propanediol, retaining water and other feed molecules (ii) allow passage of water and other molecules, retaining 1,2-propanediol or (iii) allow passage of water and 1,2-propanediol while retaining other molecules. In the present invention method (iii) is preferred. Particularly useful, are reverse osmosis membranes such as SW-30 2540 (Filmtec, Dow Chemical Co.) and the DL and SH series of reverse osmosis membranes made by Millipore (Millipore Corporation, Bedford, Mass.). Following evaporation and membrane concentration, partially purified 1,2-propanediol may be extracted into organic solvents. Suitable solvent will include alcohols such as tert-amyl alcohol, cyclopentanol, octanol, propanol, methanol, and ethanol. Non alcohols may also be used such as octanone, cyclohexane and valeraldehyde. Within the context of the present invention, alcohols are preferred and ethanol is most preferred. Alternatively 1,2-propanediol may be further concentrated by adsorption to various industrial adsorbents. Activated carbon and polycyclodextrin such as those produced by the American Maize Products Company are particularly suitable. Following either extraction or adsorption, partially purified 1,2-propanediol must be refined. Refining may be accomplished by electrodialysis (particularly useful for desalting) which utilizes a combination of anion and cation exchange membranes or biopolar (anion and cation) membranes (see for example, Grandison, Alistair S., (1996)) A preferred method of refining in the present invention is distillation. Distillation may be done in batch where the operating pressure is ambient or below, e.g. about 25 in. Hg of vacuum. Monitoring of distillation indicated that materials evaporated in the order of first to last beginning with light organics, water, diols including 1,2-propanediol and finally heavy materials such as glycerol and precipitated solids.

### EXAMPLES

The following Examples provide illustrative embodiments.

All manipulations and techniques necessary to construct and propagate strains described in this invention are known to those skilled in the art. Technical details are described e.g. in Ausubel et al 1995; Sambrook, J, 2001 and Miller, J.H. 1992 and in relevant publications cited within this invention.

### EXAMPLE 1: General Methodology

### 1.1 Strain cultivation

*E. coli* was cultured in a defined minimal medium that was designed to contain low levels of phosphate, since phosphate is a known inhibitor of methyglyoxal synthase. Per liter, the medium contained:
(NH₄)₂SO₄ - 3 g
Yeast extract - 0,2 g
CoCl₂ - 1,9 e-6 g
Bis-(2-hydroxyethyl)-imino-tris-(hydroxymethyl)-methane - 10 g
KH₂PO₄ - 0,002 g
K₂HPO₄ - 0,0085 g
MgSO₄ - 0,225 g
Trace element solution [Pfennig, 1966] - 1 ml

If appropriate, antibiotics were added to the medium. Concentrations used were gentamycin, 5 µg/l, ampicillin, 10 µg/l.

Crude glycerol was obtained from biodiesel production and had a purity of 85 %.

*E. coli* strains were routinely propagated in cultivation tubes (total volume 30 ml. Inoculum 5 ml) or glass bottles sealed with rubber-stoppers (total volume 12 ml, inoculum 10 ml) for creating semi-anoxic conditions. The term "cultivation under oxic conditions" implies cultivation in non-sealed containments with agitation. Semi-anoxic in that context means cultivation of strains without agitation in medium that was prepared under oxic conditions and in closed containments, e.g. bottles sealed with rubber-stoppers upon inoculation to avoid diffusing in of external oxygen. Cultivation times varied between 2 and 5 days, for oxic and semi-anoxic conditions, respectively. In general, experiments were stopped when optical density failed to increase further.

### 1.2 Analysis of 1,2-PD formation

Levels of 1,2-PD in supernatants in culture broth were determined by three different methods, comprising a colorimetric assay, HPLC and GC-MS. Whereas HPLC using a cation-exchange column did not allow for differentiation between 1,2- and 1,3-isomers of propanediol, the colorimetric assay was specific for 1,2-PD. GC-MS analysis allowed for simultaneous quantification of both isomers separately. Detection levels where 0,5 g/l for HPLC-analysis, 50 mg/l for the colorimetric assay, and 10 mg/l for GC-MS analysis.

For routine analysis, 1,2-PD in supernatants was determined by a colorimetric method described by Jones and Riddick {Jones, 1957}. Basically, sulphuric acid is added to cell-free supernatant sample, mixed and heated. Thereafter, a ninhydrin solution and sodium-bisulfite is added, mixed and incubated for one hour. Another aliquot of sulphuric acid is added and the absorption at 595 nm, which is equivalent to the concentration of 1,2-PD, is recorded.

For quantitative analysis, 1,2-PD in samples was measured by GC-MS analysis. 1,2-PD was identified by identical retention times compared to authentic material and by mass-fingerprinting.

### EXAMPLE 2: Construction of recombinant organisms

### 2.1 Strains and plasmids used in this invention

*E. coli* MG1655 (F- lambda- *ilvG- rfb-*50 *rph-1*) and DH5alpha (F⁻, ϕ80d*lac*ZΔM15, Δ(*lacZYA-argF*) U169, *deoR, recA*1, *endA*1, *hsdR*17(*rk*⁻, mk⁺), *phoA, supE*44, *λ⁻, thi*-1, *gyrA*96, *relA*1) and derivatives thereof were used as host for the production of 1,2-PD. Furthermore, genomic DNA of MG1655 provided the source for amplification of relevant genes. Genomic DNA from *Citrobacter freundii* (DSM30040) was used as template for the amplification of the *dhaK* gene.

### 2.2 Isolation and cloning of genes

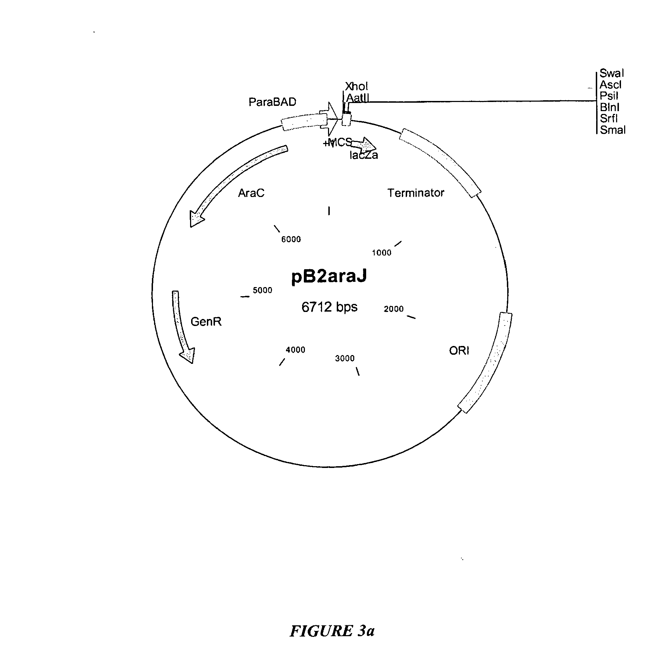

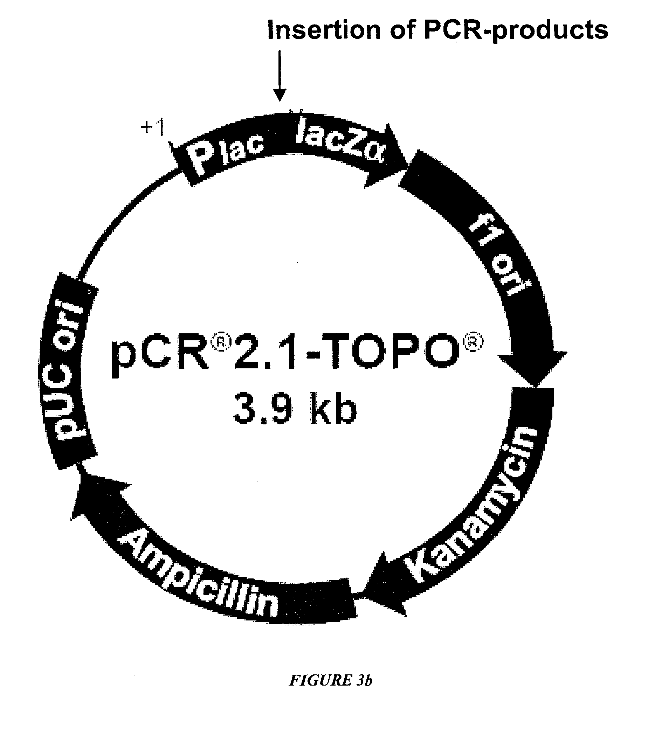

As first step, the *E. coli* gene for glycerol dehydrogenase gldA (SEQ ID NO: 29) was introduced in plasmid pB2araJ (Fig. 3a) or plasmid pCR2.1 (Fig. 3b). All primers used for amplification of genes of interest are listed in Table 1. Primers gldH_for1 and gldH_rev1 were used to amplify the 1,104-bp *gldA* Fragment from *E. coli.* The gel-purified PCR-fragment was inserted into the AatII-SwaI site of the pB2araJ vector, to give pDP_g. In this plasmid gldA-expression is under control of promotor paraBAD, allowing a tightly regulated, inducible expression by L-arabinose as described by Guzman *et al.* (Guzman, L.M., 1995). The *dhaK-*gene was amplified from *C. freundii* using the primers dhaK_for1 and dhaK_rev1. The obtained 1659-nt sequence is shown in SEQ ID NO: 27, the corresponding protein sequence in SEQ ID NO: 30. The gel-purified Fragment of dhaK was inserted into the SwaI-AscI site of pDP_g, resulting in pDP_gd, which cotranscribes both *gldA* and *dhaK.* The primers fucO_for1 and fucO_rev1 were used to amplify the 1152-nt gene encoding propanediol oxidoreductase (fucO) from *E. coli* (SEQ ID NO: 31), which was ligated into the AvrII-SmaI-site of pDP_gd, to obtain the plasmid pDP_gdf cotranscribing *gldA, dhaK and fucO.* The 468-bp fragment of mgsA encoding the *E. coli* methylglyoxalsynthase as shown in SEQ ID NO: 32 was amplified from *E. coli* using the primers mgsA_xhoI_for and mgsA_xhoI_rev and introduced in sense-orientation into the XhoI-site to obtain pDP_mgdf. The succession of genes transcribed upon induction from plasmid pDP_mgdf is thus as follows: *mgsA, gldA, dhaK, fucO and lacZ-alpha,* whereas the remaining LacZalpha-peptide was used only for transcriptional studies in a suitable host strain (e.g. DH5alpha). The nt sequence of the entire plasmid pDP_mgdf is shown in SEQ ID NO: 28.

Genes dkgA and dkgB encoding multifunctional MG-reductase (SEQ ID NO: 33) and 4-nitrobenzaldehyde reductase (SEQ ID NO: 34), respectively, were amplified from *E. coli* using Taq-polymerase and the primers dkgB_up and dkg_dw or dkgA_up and dkgA_dw. Purified PCR-products were introduced into vector pCR2.1 (Fig. 3b) by TA-cloning (Invitrogen).

**TABLE 1:**

| Primers used for PCR-amplifcation of genes to be cloned in pB2araJ or pCR2.1 | | | |
|---|---|---|---|
| Primer | Sequence (5' -........-3') | Restriction site | SEQ ID NO |
| | for cloning in pB2araJ | | |
| gldH_for1 | | AatII | 1 |
| gldH_rev1 | | SwaI | 2 |
| dhaK_for1 | | SwaI | 3 |
| dhaK_rev1 | | AscI | 4 |
| fucO_for1 | | AvrII | 5 |
| fucO_rev1 | | SmaI | 6 |
| mgsA_xhoI_for | | XhoI | 7 |
| mgsA_xhoI_rev | ATCTCGAGTTACTTCAGACGGTCCGCGA | Xhol | 8 |
| mgsAKD_for | CGCCGATTCCGGTAAAGCTG | - | 9 |
| mgsAKO_rev | GATCCTGGCGCGTTACCATC | - | 10 |
| | for cloning in pCR2.1 | | |
| dkgB_up | | - | 11 |
| dkgA_dw | TTGGCGCGCCCTTAATCCCATTCAGGAGCC | - | 12 |
| dkgA_up | TTGGCGCGCCGAATTTAAGGAATAAAGATAATGGC TAATCCAACCG | - | 13 |
| dkgA_dw | TTGGCGCGCCCTTAGCCGCCGAACTGGTCAG | - | 14 |

### 2.3 Deletion of activities encoded by gloA, gloB, rpoS, aldA, ldhA within E. coli host strains

Several techniques for specific gene-deletion are known to those skilled in the art. These techniques comprise, but are not limited to, gene disruption by modified group II introns (Karberg, M., 2001), phage-recombinase mediated gene inactivation using PCR-amplified DNA (Datsenko, K.A., 2000) (Ellis, E.H.,2001) (Yu, D., 2000) (Marx, and Lidstrom, 2002) and introducing linear double stranded DNA homologous to the gene of interest into host cells (Cunningham, R. P., et al. (1980)).

The technique preferentially used to introduce these genes into the strain is electroporation, which is well known to those skilled in the art.

In this invention, homologous recombination of PCR-amplified DNA harbouring selectable marker genes was used. Primers were specifically designed for the genes of interest. Primer sequences are listed below. Successful deletion of the gene of interest was verified by PCR-analysis and DNA-sequencing.

Denotation of deleted genes (1-6) and primers used for generation of homologous linear DNA:
1) Name: subunit of aldehyde dehydrogenase A
   Gene: *aldA*
   Accession number: Ecogene: EG10035
   Chromosomal localisation: 1486256 => 1487695
   Primer 1:
   AACAATGTATTCACCGAAAACAAACATATAAATCACAGGAGTCGCCCATG (SEQ ID NO: 15)
   Primer 2:
   GAGGAAAAAACCTCCGCCTCTTTCACTCATTAAGACTGTAAATAAACCAC (SEQ ID NO: 16)
2) Name: D-lactate dehydrogenase
   Gene: *ldhA*
   Accession number: Ecogene: EG13186
   Chromosomal localisation: 1440867 => 1439878
   Primer 1:
   CTCCCCTGGAATGCAGGGGAGCGGCAAGATTAAACCAGTTCGTTCGGGCA (SEQ ID NO: 17)
   Primer 2:
   TATTTTTAGTAGCTTAAATGTGATTCAACATCACTGGAGAAAGTCTTATG (SEQ ID NO: 18)
3) Name: RNA polymerase, sigma S (sigma 38) factor
   Gene: *rpoS*
   Accession number: Ecogene: EG10510
   Chromosomal localisation: 2865573 => 2864581
   Primer 1:
   TGAGACTGGCCTTTCTGACAGATGCTTACTTACTCGCGGAACAGCGCTTC (SEQ ID NO: 19)
   Primer 2:
   CTTTTGCTTGAATGTTCCGTCAAGGGATCACGGGTAGGAGCCACCTTATG (SEQ ID NO: 20)
4) Name: Glyoxylase I
   Gene: *gloA*
   Accession number: Ecogene: EG13421
   Chromosomal localisation: 1725861 => 1726268
   Primer 1:
   TACTAAAACAACATTTTGAATCTGTTAGCCATTTTGAGGATAAAAAGATG (SEQ ID NO: 21)
   Primer 2:
   GGCGCGATGAGTTCACGCCCGGCAGGAGATTAGTTGCCCAGACCGCGACC (SEQ ID NO: 22)
5) Name: Glyoxylase II
   Gene: *gloB*
   Accession number: Ecogene: EG13330
   Chromosomal localisation: 234782 => 234027
   Primer 1:
   CGAACGGAGCCGATGACAAGAAAGTTTTATCAGAACCTATCTTTCTTTGA (SEQ ID NO: 23)
   Primer 2:
   CTTGCCGGTTTCATCACAACCTTCCGTTTCACACTGAGAGGTAATCTATG (SEQ ID NO: 24)
6) Name: L-ribulokinase monomer
   Gene: *araB*
   Accession number: Ecogene: EG10053
   Chromosomal localisation: 70048 => 68348
   Primer 1:
   AATTATCAAAAATCGTCATTATCGTGTCCTTATAGAGTCGCAACGGCCTG (SEQ ID NO: 25)
   Primer 2:
   ACTCTCTACTGTTTCTCCATACCCGTTTTTTTGGATGGAGTGAAACGATG (SEQ ID NO: 26)

### EXAMPLE 3: 12-PD Production

### 3.1 Culturing of microorganisms in crude-glycerol-minimal medium

Utilisation of preparations of crude glycerol (purity about 85 %) compared to essentially pure preparations of glycerol (purity > 99%) by different microorganisms was investigated. A broad variety of microorganims representig different taxa along with *E. coli* MG1655 were grown at unregulated pH in minimal medium supplemented with different amounts of pure and crude glycerol under oxic conditions. Tab. 2 and Tab. 3 demonstrate that no inhibitory effect on biomass production was observed when crude preparation of glycerol instead of pure glycerol was the sole source of carbon and energy. Furthermore, the amount of phosphate in a defined minimal medium can be reduced by 70 % when crude glycerol served as source for carbon and energy (Tab. 4).

**TABLE 2:**

| | | |
|---|---|---|
| Biomass-production sustained by crude-glycerol preparations. Biomass production was compared when pure (purity >99%) or crude preparations of glycerol served as carbon source (10 g/l) for growth under oxic or anoxic condtions. Strains representing different taxa isolated from environmental samples were cutlivated in microtiterplates without agitation under atmospheric conditions specified. | | |

| | | biomass production by crude glycerol equal or higher compared to pure preparations of glycerol |
|---|---|---|
| | Total no. | [%] |
| isolates tested | 374 | - |
| oxic conditions | 304 | 91,3 |
| anoxic condtions | 369 | 98,7 |

**TABLE 3:**

| | | | | |
|---|---|---|---|---|
| Comparison of biomass-production of *E. coli* MG1655 obtained by crude- or pure preparations of glycerol. Biomass production was compared when pure (purity >99%) or crude preparations of glycerol served as carbon source for growth under oxic conditions at unregulated pH. Strains were cutlivated in cultivation tubes under oxic conditions Ø, average; stdev, standard deviation | | | | |

| | **Biomass production [OD580]** | | | |
|---|---|---|---|---|
| **substrate** | **crude glycerol** | | **pure glycerol** | |
| **]g/l]** | **Ø** | **stdv** | **Ø** | **stdv** |
| **0,63** | **0,3** | 0,09 | **0,5** | 0,09 |
| **1,25** | **0,6** | 0,16 | **0,6** | 0,00 |
| **2,5** | **1,0** | 0,16 | **1,2** | 0,00 |
| **10** | **4,7** | 0,10 | **5,0** | 0,33 |
| **20** | **5,9** | 0,41 | **6,0** | 0,75 |
| **40** | **7,1** | 1,84 | **5,7** | 0,82 |

**TABLE 4:**

| | | | | |
|---|---|---|---|---|
| Potential of impurities present in crude glycerol (10 g/l) to substitute for macro-elements of minimal medium. Biomass-production *of E. coli* MG1655 was compared when pure (purity >99%) or crude preparations of glycerol served as source for carbon and macro-elements for growth under oxic condtions at unregulated pH. Ø, average; stdev, standard deviation | | | | |

| | Biomass production [OD580] | | | |
|---|---|---|---|---|
| cultue broth | crude glycerol | | pure glycerol | |
| devoid of | Ø | stdev | Ø | stdev |
| none | 4,6 | 0,75 | 2,5 | 0,34 |
| nitrogen | 1,0 | 0,00 | 1,1 | 0,09 |
| phosphate | 3,2 | 0,75 | 1,2 | 0,16 |
| sulfate | 1,5 | 0,57 | 1,0 | 0,16 |
| trace-elements | 2,9 | 0,34 | 2,4 | 0,33 |

### 3.2 Constitution of a functional pathway yielding dihydroxyacetone phosphate bypassing glycerol kinase and glyceraldehyde-3-phosphate dehydrogenase

Genes gldA from *E. coli* encoding glycerol dehydratase, dhaK from Citrobacter freundii encoding dihydroxyacetone kinase (dhaK) and fucO from *E. coli* encoding propanediol-oxidoreductase were cloned in plasmid pB2araJ to give plasmid pDP_gdf. Plasmid pDP_mgdf additionally contains methylglyoxal synthase from E. coli. According to known biochemical pathways, propanediol oxidoreductase is not relevant for this example. However, we tested plasmid pDP_gdf and its derivative, pDP_mgdf for functional complementation of a glpK-knock out, since these plasmids are relevant for recombinant 1,2-PD production.

The assay demonstrated that the presence of plasmid pDP_gdf or pDP_mgdf relieved inhibition of growth in a glpK-mutant of *E. coli* when glycerol was the sole carbon source (Tab. 5). Thus, an inducible unregulated pathway independent of the endogenous route to metabolise glycerol was established. The biomass finally obtained is equal or even higher compared to growth of a control strain (DH5alphadeltaaraB) or when glucose is the substrate for growth.

**TABLE 5:**

| | | | | | | | |
|---|---|---|---|---|---|---|---|
| An araB-knockout of *E. coli* DH5alpha and derivatives thereof were cultivated in minimal medium containing glucose or glycerol as carbon source at 10 g/l. The different strains were cultivated at 37°C in cultivation tubes under oxic conditions for 5 days. Biomass was determined as optical density at 580 nm at the end of the expriment. Stdev, standard deviation | | | | | | | |

| | | | | Biomass production Carbon source | | | |
|---|---|---|---|---|---|---|---|
| | Genes inacitvated | | Plasmid | Glucose | | Glycerol | |
| E.coli strain | | | pDP_ | OD580 | stdev | OD580 | stdev |
| DH5alpha | araB | - | - | 3,7 | - | 2,0 | 0,1 |
| DH5alpha | araB | glpK | - | 3,0 | - | 0,1 | 0,0 |
| DH5alpha | araB | glpK | gdf | 2,7 | - | 4,5 | 0,2 |
| DH5alpha | araB | glpK | mgdf | 5,6 | 0,2 | 5,5 | 0,3 |

### 3.3 Defining a minimal set of genes indispensible for recombinant 1,2-PD production, from glycerol

An araB mutant of *E. coli* DH5alpha was transformed with different plasmids containing genes of interest listed in Table 6. The wild-type (control) and recombinant strains were cultivated under oxic conditions in minimal medium containing 10 g/l crude glycerol for 3 - 5 days until the strains entered stationary phase. Supernatants were analysed by GC-MS analysis for 1,2-PD contents. Results are given in table 6.

**TABLE 6:**

| 1,2-PD contents determined in supernatants upon cultivation of *E. coli* strains in minimal medium containing 10 g/l of crude glycerol. | | | | | |
|---|---|---|---|---|---|
| | | | | 1,2-PD | stdev |
| E. coli strain | genes inactivated | plasmids | genes expressed | [mg/l] | [mg/l] |
| DH5a | araB | none | - | 0 | 0 |
| DH5a | araB | pB2araJ | - | 0 | 0 |
| DH5a | araB | pDP_g | gldA | 0 | 0 |
| DH5a | araB | pDP_f | fucO | 39 | 4 |
| DH5a | araB | pDP_gd | gldA, dhaK | 0 | 0 |
| DH5a | araB | pup_gd | gldA, dhaK, mgsA | 0 | 0 |
| | | pUC_mgsA | | | |
| DH5a | araB | pDP_gdf | gldA, dhaK, fucO | 40 | 3 |
| DH5a | araB | pDP_gdf | gldA, dhaK, fucO, mgsA | 59 | 9 |
| | | pUC_mgsA | | | |

### 3.4 Exclusive production of the 1,2-isomer of propanediol by recombinant E.coli strains expressing glycerol dehydrogenase, dihydroxyacetone kinase., methylglyoxal synthase and propanediol oxidoreductase encoded in a synthetic operon

*E. coli* MG1655 deltaaraB was transformed with or without plasmid pDP_mgdf and cultivated in minimal-medium containing 10 or 15 g/l crude glycerol as carbon source. Incubation was done under oxic conditions in cultivation tubes, or in sealed glass vials without agitation (semi-anoxic conditions). *E. coli* MG1655deltaaraB without plasmid was the control strain. Incubation period was 5 days, incubation temperature was 37°C.

At the end of the experiment, growth was determined as optical density (OD580), and 1,2- or 1,3-PD levels were determined by GC-MS analysis. Assays were done in duplicate. The results (Tab. 7) demonstrate successful introduction of an engineered pathway that enables *E. coli* to produce 1,2-PD from crude glycerol that produces exclusively the 1,2-isomer of propanediol.

**TABLE 7:**

| | | | | | | | | |
|---|---|---|---|---|---|---|---|---|
| araB-knock out stains of *E. coli* MG1655 were transformed with/without plasmid pDP_mgdf and cultivated in minimal medium containing crude glycerol as carbon source at concentrations of 10 or 15 g/l. Cultivation was done at 37°C under oxic and semi-anoxic conditions. Biomass was determined as optical density at 580 nm at the end of the experiment (5 days); 1,2-PD contents in the supernatant were determined by GC-MS analysis; 0, not detected, detection-limit 10 mg/l. Ø, average; stdev, standard deviation | | | | | | | | |

| | | | | substrate | propanediol production | | | |
|---|---|---|---|---|---|---|---|---|
| | | | | | 1,2-PD [mg/l] | | 1,3-PD [mg/l] | |
| *E. coli* strain | genes inactivated | plasmids | cultivation | crude glycerol [g/l] | Ø | stdev | Ø | stdev |
| MG1655 | araB | none | oxic | 10 | 0 | 0 | 0 | 0 |
| MG1655 | araB | none | oxic | 15 | 0 | 0 | 0 | 0 |
| MG1655 | araB | pDP_mgdf | oxic | 10 | 203 | 5 | 0 | 0 |
| MG1655 | araB | pDP_mgdf | oxic | 15 | 435 | 11 | 0 | 0 |

### 3.5 Toxicity of methylglyoxal to wild-type and Mutant strains of E. coli

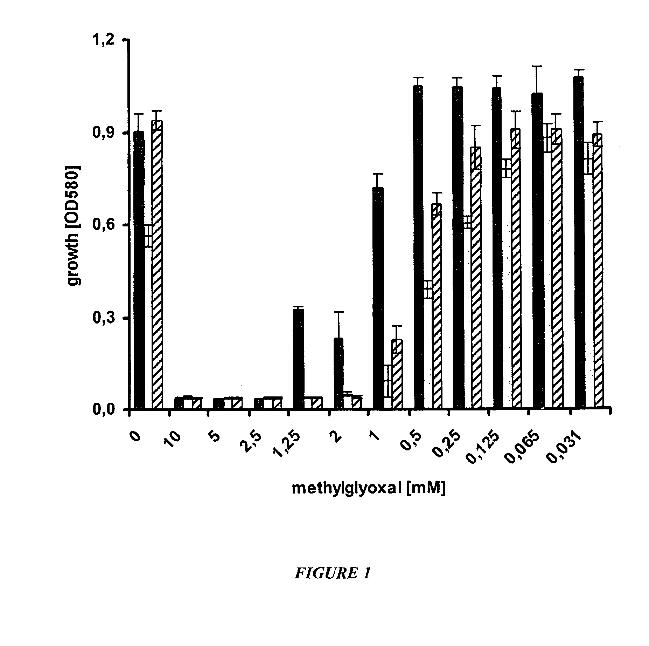

*E. coli* MG1655 wild-type or mutants inactivated in the genes gloA and gloB, respectively, were cultivated in minimal-medium with pure-glycerol (10g/l) as source for carbon and energy containing different amounts of methylglyoxal. Tests for methylglyoxal indicated a period of at least 72 h of chemical stability. *E*. *coli* was cultivated in microtiterplates without agitation at 37 °C under a humid atmosphere. Growth was determined at OD580 48,5h after inoculation (Fig. 1; black bars, E. coli MG1655 wild-type; grey bars, E. coli MG1655 gloA-mutant; hatched bars, E. coli MG1655 gloB-mutant). Inhibition of growth of the wild-type strain was observed for extracellular concentration of methylglyoxal equal or higher than 2,5 mM. The mutant strains were significantly more sensitive, especially the glyoxylase I mutant (gloA), thus substantiating our finding, that gloA is a major drainage pathway for intracellular methylglyoxal (see 4.6). The results furher demonstrate that elevated levels of methylglyoxal inhibit growth of *E. coli* which is a basis for the identification of phosphate-insensitive variants of methylglyoxal-synthases.

### 3.6 Identification of major MG-withdrawing activity

*E. coli* MG1655 was inactivated (gene knockout) in the genes whose gene products are involved in methylglyoxal (MG) metabolism, e.g. detoxification of MG. The genes are listed in table 8. Wild-type and mutant strains were cultivated in cultivation tubes under oxic conditions and agitation until growth ceased. Levels of 1,2-PD in the bulk liquid were determined by GC-MS analysis and compared to levels that were found when glucose (negative control) was source for carbon and energy. When glycerol was substrate for growth, a background level of about 20 mg/ml 1,2-PD was detected. However, significantly elevated 1,2-PD levels were observed for the gloA-mutant, solely.

**TABLE 8:**

| | | | | | | | |
|---|---|---|---|---|---|---|---|
| Strains of *E. coli* MG1655 mutated in genes involved in methylglyoxal metabolism were cultivated in minimal medium containing glycerol or glucose (10 g/l). Cultivation was performed under oxic conditions at 37°C until growth ceased. Growth was determined as optical density (OD580); Propanediol levels were determined by GC-MS analysis. ∅, average; stdev, standard deviation | | | | | | | |

| **E. coli** | | **1,2-PD [mg/l]** | | **1,3-PD [mg/l]** | | **OD 580** | |
|---|---|---|---|---|---|---|---|
| **strain** | **gene(s) inactivated** | Ø | stdev | Ø | stdev | Ø | stdev |
| MG 1655 | none | **20** | 1 | **0** | 0 | **2,6** | 0,2 |
| MG 1655 | none | **0** | 0 | **0** | 0 | **2,1** | 0,1 |
| | | | | | | | |
| MG 1655 | gloB | **33** | 1 | **0** | 0 | **2,6** | 0,2 |
| MG 1655 | gloB | **0** | 0 | **0** | 0 | **2,1** | 0,1 |
| | | | | | | | |
| MG 1655 | IdhA | **22** | 1 | **0** | 0 | **2,7** | 0,3 |
| MG 1655 | IdhA | **0** | 0 | **0** | 0 | **2,2** | 0,3 |
| | | | | | | | |
| MG 1655 | rpoS | **24** | 0 | **0** | 0 | **2,7** | 0,4 |
| MG 1655 | rpoS | **0** | 0 | **0** | 0 | **1,9** | 0,1 |
| | | | | | | | |
| MG 1655 | gloA | **113** | 5 | **0** | 0 | **2,6** | 0,2 |
| MG 1655 | gloA | **3** | 5 | **0** | 0 | **1,9** | 0,2 |
| | | | | | | | |
| MG 1655 | aldA | **25** | 1 | **0** | 0 | **2,7** | 0,1 |
| MG 1655 | aldA | **0** | 0 | **0** | 0 | **2,3** | 0,1 |
| | | | | | | | |
| MG 1655 | aldA, gloA | **102** | 6 | **0** | 0 | **1,6** | 0,0 |
| MG 1655 | aldA, gloA | **0** | 0 | **0** | 0 | **1,8** | 0,3 |
| | | | | | | | |
| MG 1655 | aldA, IdhA, gloA | **79** | 9 | **0** | 0 | **2,7** | 0,3 |
| MG 1655 | aldA, IdhA, gloA | **0** | 0 | **0** | 0 | **2,5** | 0,3 |
| | | | | | | | |
| MG 1655 | aldA, rpoS, gloA | **110** | 0 | **0** | 0 | **2,8** | 0,0 |
| MG 1655 | aldA, rpoS, gloA | **0** | 0 | **0** | 0 | **2,5** | 0,8 |

### 3.7 Recombinant organisms producing high levels of 1,2-PD

*E. coli* MG1655 was inactivated (gene knockout) in the genes araB, or in genes aldA and gloA. Corresponding mutants were transformed with plasmid pDP_mgdf. AraB-mutants harbouring plasmid pDP_mgdf were additionally transformed with plasmid pCR2.1 encoding genes conferring aldo-keto reductase activity, e.g. dkgA or dkgB of *E. coli.* Cells were cultivated in presence of crude-glycerol (15 g/l) under oxic or semi-anoxic conditions at 37°C for 2 - 5 days or until optical density failed to increase further. Supernatants were analysed for 1,2-PD content by GC-MS analysis (Tab. 9).

**TABLE 9:**

| | | | | | | | | |
|---|---|---|---|---|---|---|---|---|
| Mutant strains of *E. coli* MG1655 expressing glycerol-to-1,2-PD converting genes were cultivated in minimal medium containing glycerol or glucose (15 g/l). Cultivation was performed under oxic or semi-anoxic conditions at 37°C until growth ceased. Propanediol levels in supernatants were determined by GC-MS analysis. ∅, average; stdev, standard deviation | | | | | | | | |

| **E. coli strain** | **gene(s) inactivated** | **plasmids present** | | **cultivation** | **1,2-PD [mg/l]** | | **1,3-PD [mg/l]** | |
|---|---|---|---|---|---|---|---|---|
| | | plasmid 1 | plasmid 2 | | **∅** | stdev | **∅** | stdev |
| MG 1655 | aldA, gloA | none | none | oxic | 65 | 5 | 0 | 0 |
| MG 1655 | aldA, gloA | none | none | semi-anoxic | 30 | 0 | 0 | 0 |
| | | | | | | | | |
| MG 1655 | aldA, gloA | pDP_mgdf | none | oxic | 340 | 60 | 0 | 0 |
| MG 1655 | aldA, gloA | pDP_mgdf | none | semi-anoxic | 370 | 20 | 0 | 0 |
| | | | | | | | | |
| MG 1655 | araB | pDP_mgdf | pCR_dkgA | oxic | 440 | 110 | 0 | 0 |
| MG 1655 | araB | pDP_mgdf | pCR_dkgA | semi-anoxic | 260 | 0 | 0 | 0 |
| | | | | | | | | |
| MG 1655 | araB | pDP_mgdf | pCR_dkgB | oxic | 275 | 15 | 0 | 0 |
| MG 1655 | araB | pDP_mgdf | pCR dkgB | semi-anoxic | 340 | 170 | 0 | 0 |

### RESULTS

As shown in table 2 and 3, crude preparations of glycerol can be utilised without further processing by a wide variety of organisms not restricted to *E. coli* and close relatives. Biomass production obtained by utilizing crude glycerol is equal to or higher when compared with pure glycerol as carbon substrate. Thus, crude-glycerol substitutes for pure preparations of glycerol in virtually any process that is based on the fermentation of glycerol.

As shown in table 4, impurities present in crude-glycerol provide a source of phosphor sustaining substantial growth of host cells. Thus, the amount of phosphor added to the culture broth can be decreased by about 70%.

As shown in table 6, propanediol oxidoreductase activity is indispensible for the synthesis of 1,2-PD from glycerol. Host cells that express glycerol-dehydrogenase do not produce 1,2-PD. Cells that express glycerol-dehydrogenase and methylglyoxal-synthase but lacking propanediol-oxidoreductase do not produce detectable amounts of 1,2-PD, whereas additional presence of propanediol-oxidoreductase activity results in significant 1,2-PD production.

As shown in table 7, high titers of 1,2-PD are obtained from glycerol with recombinant strains coexpressing methylglyoxal-synthase, glycerol-dehydrogenase, dihydroxyacetone-kinase and propanediol-oxidoreductase under oxic conditions. The synthesis of propanediol based on the present invention results in the synthesis of exclusively the 1,2-isomer but not the 1,3-isomer of propanediol.

As shown in table 8, inactivation of glyoxylase I activity encoded by gloA in *E. coli* results in significant production of 1,2-PD from glycerol. Thus, enzyme activitiy encoded by gloA is the major competing activity interfering with high-level production of 1,2-PD in host ells.

Further described herein are the following items:
1. A host cell engineered to produce high levels of 1,2-propanediol when grown on glycerol as the sole carbon source.
2. A host cell according to the preceding item, wherein the glycerol has a degree of purity of at least 70%, particularly of at least 75%, particularly of at least 80%, particularly of at least 85%, particularly of at least 90%, particularly of at least 95%, particularly of at least 99% and up to 100%.
3. A host cell according to any of the preceding items, wherein the glycerol has a degree of purity of between 80% and 90%.
4. A host cell according to any of the preceding items, wherein the glycerol has a degree of purity of about 85%.
5. A host cell according to any of the preceding items, wherein the glycerol is a crude glycerol preparation from biodiesel and/or bioethanol production.
6. A host cell according to any of the preceding items wherein said host cell has been engineered through recombinant DNA techniques.
7. A host cell according to any of the preceding items, particularly to item 6, wherein said host cell has been engineered by introducing a gene encoding a propanediol oxidoreductase activity (fucO).
8. A host cell according to item 7, wherein said host cell has been engineered by introducing at least one additional gene encoding an enzyme activity selected from the group consisting of glycerol dehydrogenase (gldA), dihydroxyacetone kinase (dhaK) and methylglyoxalsynthase (mgsA) such as to express said activities along with the propanediol oxidoreductase activity (fucO).
9. A host cell according to item 7, wherein said host cell has been engineered by introducing an additional gene encoding a glycerol dehydrogenase such as to express said glycerol dehydrogenase activity along with the propanediol oxidoreductase activity.
10. A host cell according to item 7, wherein said host cell has been engineered by introducing an additional gene encoding a dihydroxyacetone kinase such as to express said dihydroxyacetone kinase activity along with the propanediol oxidoreductase activity.
11. A host cell according to item 7, wherein said host cell has been engineered by introducing an additional gene encoding a methylglyoxalsynthase (mgsA) such as to express said methylglyoxalsynthase activity along with the propanediol oxidoreductase activity.
12. A host cell according to item 7, wherein said host cell has been engineered by introducing additional genes encoding a glycerol dehydrogenase and a dihydroxyacetone kinase such as to express said glycerol dehydrogenase and dihydroxyacetone kinase activities along with the propanediol oxidoreductase activity.
13. A host cell according to item 7, wherein said host cell has been engineered by introducing additional genes encoding a glycerol dehydrogenase and a methylglyoxalsynthase such as to express said glycerol dehydrogenase and methylglyoxalsynthase activities along with the propanediol oxidoreductase activity.
14. A host cell according to item 7, wherein said host cell has been engineered by introducing additional genes encoding a dihydroxyacetone kinase and a methylglyoxalsynthase such as to express said dihydroxyacetone kinase and methylglyoxalsynthase activities along with the propanediol oxidoreductase activity.
15. A host cell according to item 7, wherein said host cell has been engineered by introducing additional genes encoding a glycerol dehydrogenase, a dihydroxyacetone kinase and a methylglyoxalsynthase such as to express said glycerol dehydrogenase, dihydroxyacetone kinase and methylglyoxalsynthase activities along with the propanediol oxidoreductase activity.
16. A host cell according to any of the preceding items, particularly to item 7, wherein said host cell has been engineered by introducing additional genes encoding a glycerol dehydratase such as to express said glycerol dehydratase activity along with the propanediol oxidoreductase activity.
17. A host cell according to any of the preceding items, particularly to item 7, wherein said host cell has been engineered by introducing additional genes encoding an aldo-keto-reductase such as to express said aldo-keto-reductase activity along with the propanediol oxidoreductase activity.
18. A host cell according to item 17, wherein said aldo-keto-reductase activity is contributed by a gene selected from the group consisting of dkgA, dkgB, yeaE and yghZ.
19. A host cell according to any of the preceding items, wherein said host cell is defective in arabinose metabolism.
20. A host cell according to the preceding item, wherein said defect is due to a reduced or missing ribulose kinase activity.
21. A host cell according to any of the preceding items, wherein said host cell is defective in the metabolism of methylglyoxal.
22. A host cell according to the preceding item, wherein said defect is due to a reduced or missing enzyme activity selected from the group consisting of glyoxylase system I, glyoxylase system II, lactate dehydrogenase A, glyoxylase system III, aldehyde dehydrogenase A activity, but particularly a glyoxylase system I activity.
23. A host cell according to any of the preceding items, wherein said host cell is defective in the metabolism of dihydroxyacetonphosphate.
24. A host cell according to the preceding item, wherein said defect is due to a reduced or missing triosephosphate isomerase activity.
25. A host cell according to any of the preceding items, which produces high levels of 1,2-propanediol when grown on glycerol as the sole carbon source, but essentially no 1,3-propanediol.
26. A host cell according to any of the preceding items, which is a microbial or a fungal host cell.
27. A microbial host cell according to item 26, which is *E. coli.*
28. A polynucleotide molecule comprising a synthetic operon under the control of an inducible promoter, which operon comprises the genes encoding glycerol dehydrogenase (gldA) and propanediol oxidoreductase (fucO).
29. A polynucleotide molecule comprising a synthetic operon under the control of an inducible promoter, which operon comprises the genes encoding glycerol dehydrogenase (gldA), dihydroxyacetone kinase (dhaK) and propanediol oxidoreductase (fucO).
30. A polynucleotide according to item 29 wherein the synthetic operon is extended to further contain a gene encoding methylglyoxal synthase (mgsA).
31. A polynucleotide according to item 29 and 30, wherein the genes encoding glycerol dehydrogenase (gldA); propanediol oxidoreductase (fucO) and methylglyoxal synthase (mgsA), respectively, are obtainable from *E. coli* and the gene encoding dihydroxyacetone kinase (dhaK) is obtainable from *C freundii.*
32. A polynucleotide according to any of the preceding items, which is a plasmids.
33. A polynucleotide according to any of the preceding items wherein the arrangement of the genes in the synthetic operon is 5'-mgsA-gldA-dahK-fuc0-3'.
34. A polynucleotide according to any of the preceding items wherein the inducible promoter is an arabinose-inducible promoter.
35. A microorganism according to any of the preceding items comprising a polynucleotide according to anyone of items 28 to 34.
36. A microorganism according to any of the preceding items comprising a phosphate-insentive mgsA gene, which is fully operable under high phosphate concentrations, particularly under phosphate concentrations higher than 0,7 mM in the cultivation medium or higher than 9,3e-05 in the cytoplasm of the cell.
37. A method for the production of 1,2-propanediol comprising growing a host cell according to any one of items 1 to 27 in an appropriate growth medium containing a simple carbon source, particularly a crude glycerol preparation, after which the 1,2-propanediol produced are recovered and, optionally, purified.
38. A method according to item 37, comprising: items
   i) culturing a host cell according to any one of items 1 to 27, which host cell overexpresses propanediol oxidoreductase (fucO) activity, in a medium containing a non-fermentable carbon substrate, whereby the carbon substrate is sustaining production of biomass and serves as a substrate for production of 1,2 propanediol (1,2-PD) at the same time, and the non-fermentable carbon source is metabolized by the host cell into 1,2-propanediol;
   ii) recovering the 1,2-propanediol produced according to step i); and, optionally,
   iii) purifying the recovered 1,2-propanediol.
39. A method according to any of the preceding items, wherein said non-fermentable carbon substrate is a crude glycerol preparation, particularly a preparation containing glycerol with a purity of at least 70%, particularly of at least 75%, particularly of at least 80%, particularly of at least 85%, particularly of at least 90%, particularly of at least 95%, particularly of at least 99% and up to 100%.
40. A method according to any of the preceding items, wherein the glycerol has a degree of purity of between 80% and 90%, particularly of about 85%.
41. A method according to any of the preceding items, wherein the non-fermentable carbon substrate, particularly the crude glycerol preparation is selectively metabolized to 1,2-propanediol.
42. A method according to any of the preceding items, wherein a host cell is used, which is engineered to overexpress propanediol oxidoreductase (fucO).
43. A method according to any of the preceding items, wherein a host cell is used, which is engineered to co-express at least one enzyme selected from the group consisting of glycerol dehydrogenase (gldA), dihydroxyacetone kinase (dhaK) and methylglyoxalsynthase (mgsA) along with the propanediol oxidoreductase (fucO) activity.
44. A method according to any of the preceding items, wherein a host cell is used, wherein at least one enzyme activity involved in a non-productive pathway competing with 1,2-PD production has been deactivated.
45. A method according to any of the preceding items, wherein a microbial mutant, particularly a mutant of *E.coli,* is used, where one or more of the genes encoding glyoxylase systems I and II (gloA and gloB), lactate dehydrogenase A (ldhA), glyoxylase system III (indirectly by inactivation of the master regulator rpoS), and aldehyde dehydrogenase have been deactivated.
46. A method according to any of the preceding items, wherein a microbial mutant or strain, particularly an *E. coli* mutant, is used where the gene encoding a gloA activity has been partially or fully inactivated:
47. A method according to any of the preceding items, wherein a microbial mutant or strain inactivated in arabinose metabolism is used.
48. A method according to any of the preceding items, wherein an *E.coli* strain is used as the host organism, particularly an *E. coli* strain MG1655 and DH5alpha, respectively.
49. A method according to any of the preceding items, wherein at least one of the genes encoding an enzyme activity selected from the group consisting of glycerol dehydrogenase (gldA), dihydroxyacetone kinase (dhaK) and methylglyoxalsynthase (mgsA) and propanediol oxidoreductase (fucO) is under the control of an inducible promoter, particularly an arabinose inducible promoter, particularly a paraBAD promoter.
50. A method according to any of the preceding items, wherein a synthetic operon is used in the method according to the invention to provide a host cell coexpressing at least one enzyme activity selected from the group consisting of glycerol dehydrogenase (gldA), dihydroxyacetone kinase (dhaK) and methylglyoxalsynthase (mgsA) activity along with the propanediol oxidoreductase (fucO) activity.
51. A method according to any of the preceding items, wherein the genes encoding the above activities are under control of an inducible promoter, particularly an arabinose-inducible promoter, but especially a paraBAD promoter.
52. A method according to any of the preceding items, wherein the succession of genes transcribed upon induction from said operon is as follows: *mgsA, gldA, dhaK, fucO.*
53. A method for the preparation of a host cell that can be used in a method according to any one items 37 to 52 for the production of 1,2-propanediol comprising transforming said host cell with a polynucleotide according to any one of items 27 to 33.
54. A method according to item 53, wherein said host cell is a microbial host cell, particularly *E. coli.*
55. A method according to item 53, wherein transformation is accomplished by electroporation. items
56. A host cell produced by a method according to any one of items 53 to 55.

### REFERENCE LIST

Altaras, N.E, 2001; Biotechnology Progress (17), 52-56
Appleyard, R. K., Genetics, 39, 440 - 452 (1954)
Ausubel, Current Protocols in Molecular Biology, Green Publishing Associates and Wiley Interscience, N.Y. (1994).
Ausubel, F. M., Brent, R., Kingston, R. E., Moore, D. D., Seidman, J. G., Smith, J. A. & Struhl, K. (1995). Short Protocols in Molecular Biology. A Compendium of Methods from Current Protocols in Molecular Biology, 3 edn. New York: John Wiley & Sons, Inc.
Batzer, et al., Nucleic Acid Res. 19:5081 (1991);
Cameron, D.C., 1986; Bio/Technology (4), 651-654
Cameron, D.C, 1998; Biotechnology Progress (14), 116-125
Cunningham, R. P., DasGupta, C., Shibata, T., and Radding, C. M. (1980) Cell 20, 223-235).
Datsenko, K.A., 2000; PNAS (97), 6640-6645
Ellis, E.H.,2001; PNAS (98), 6742-6746
Gough JA, Murray NE., J. Mol. Biol., 166, 1 - 19 (1983)
Grandison, Alistair S., Sep. Processes Food Biotechnol. Ind. (1996), 155 177.
Guzman, L.M., 1995; Journal of Bacteriology (177), 4121-4130
Hanahan J., et al.,. Mol. Biol., 166, 557 - 580 (1983)
Heath, E.C., 1962; The Journal of Biological Chemistry (237), 2423-2426
Hopper, D.J. 1972; Biochemical Journal (128), 321-329
Jones, L. R. & Riddick, J. A. (1957). Colorimetric determination of 1,2-propanediol and related compounds. Anal Chem 39, 1214-1216.
Joyner, A. 1999, Gene Targeting, A Practical Approach, Oxford University Press. ISBN-13: 978-0-19-963792-8.
Karberg, M., 2001; Nature Biotechnology (19), 1162-1167
**Kluyver** and Schellen, 1937
Le Mouellic, K. et al, Proc. Natl. Acad. Sci. USA, 87 (1990), 4712-4716
Marx, C.J. and Lidstrom, M.E., (2002), BioTechniques (33)5, 1062-1067
Miller, J. H. (1992). A Short Course in Bacteria Genetics: A Laboratory Manual and Handbook for Escherichia Coli and Reflated Bacteria, 1st edn: Cold Spring Harbor Laboratory Press.
Neidhardt et al. 1996, Escherichia coli and Salmonella: Cellular and Molecular Biology, ASM Press
Ohtsuka, et al., J. Biol. Chem. 260:2605-2608 (1985)
Pfennig, N. & Lippert, K. D. (1966). Uber das Vitamin B12-Bedürfnis phototropher Schwefelbakterien. Archives of microbiology 55, 245-256.
Reynolds, A. B. et al. Cell 38, 275-285 (1984).
Rossolini, et al., Mol. Cell. Probes 8:91-98 (1994)
Sambrook et al., Molecular Cloning: A Laboratory Manual, Second Edition (1989) Cold Spring Harbor Laboratory Press, Cold Spring Harbor, N.Y. (1989)
Sambrook, J. & Russell, D. W. (2001). Molecular- Cloning: A Laboratory Manual, 3rd edn: Cold Spring Harbor Laboratory Press.
Tran Din, K, 1985; Archives of Microbiology (142), 87-92
Wood, W.B., J. Mol. Biol., 16, 118 - 133 (1966)
Yu, D., 2000; PNAS (97), 5978-5983
Jerpseth, B. et al. Strategies, 5, 81-83 (1992)
Young, R.A. and R.W. Davis, Science, 222, 778 - 782 (1983)

### Patent Literature

| | |
|---|---|
| U.S. Pat. No. 5,008,473 | U.S. Pat. No. 4683202 |
| U.S. Pat. No. 5,356,812 | US Pat. Appl. 2007/072279 |
| U.S. Pat. No.7049109 | WO 2005/073364 |
| U.S. Pat. No. 6087140 | |
| U.S. Pat. No. 6303352 | |

## Claims

1. A host cell engineered to produce high levels of 1,2-propanediol when grown on glycerol as the sole carbon source, wherein said host cell has been engineered by introducing a gene encoding a propanediol oxidoreductase activity (fucO), a gene encoding a glycerol dehydrogenase (gldA) and a gene encoding dihydroxyacetone kinase (dhaK) and wherein the gene encoding the dihydroxyacetone kinase activity is isolated from a Citrobacter strain.

2. A host cell according to claim 1, wherein the glycerol has a degree of purity of between 80% and 90%.

3. A host cell according to claim 1, wherein said host cell has been engineered by introducing an additional gene encoding a methylglyoxalsynthase (mgsA) such as to express said activity along with the propanediol oxidoreductase activity, the glycerol dyhydrogenase activity and the dihydroxyacetone kinase activity.

4. A host cell according to claim 1, wherein said host cell has been engineered by introducing additional genes encoding a glycerol dehydratase such as to express said glycerol dehydratase activity along with the propanediol oxidoreductase activity, the glycerol dyhydrogenase activity and the dihydroxyacetone kinase activity.

5. A host cell according to claim 1, wherein said host cell has been engineered by introducing additional genes encoding an aldo-keto-reductase such as to express said aldo-keto-reductase activity along with the propanediol oxidoreductase activity, the glycerol dehydrogenase activity and the dihydroxyacetone kinase activity.

6. A host cell according to any of the preceding claims, wherein said host cell is defective in at least the metabolism of compounds selected from the group consisting of:
i) arabinose
ii) methylglyoxal
iii) dihydroxyacetonphosphate.

7. A host cell according to any of the preceding claims, which produces high levels of 1,2-propanediol when grown on glycerol as the sole carbon source, but essentially no 1,3 -propanediol.

8. A host cell according to claim 7, which is E. coli.

9. A method for the production of 1,2-propanediol comprising growing a host cell according to any one of claims 1 to 8 in an appropriate growth medium containing glycerol, after which the 1,2-propanediol produced is recovered and, optionally, purified.

## Patentansprüche

1. Wirtszelle, die verändert wurde, um große Mengen von 1,2-Propandiol herzustellen, wenn sie auf Glycerin als einziger Kohlenstoffquelle gezüchtet wird, wobei die Wirtszelle durch Einführung eines Gens, das eine Propandiol-Oxidoreduktase-Aktivität (fucO) kodiert, eines Gens, das eine Glycerin-Dehydrogenase (gldA) kodiert und eines Gens, das Dihydroxyaceton-Kinase (dhaK) kodiert, verändert wurde und wobei das Gen, das die Dihydroxyaceton-Kinase-Aktivität kodiert, aus einem Citrobacter-Stamm isoliert ist.

2. Wirtszelle nach Anspruch 1, wobei das Glycerin einen Reinheitsgrad zwischen 80% und 90% hat.

3. Wirtszelle nach Anspruch 1, wobei die Wirtszelle durch Einführung eines zusätzlichen Gens verändert wurde, das eine Methylglyoxal-Synthase (mgsA) kodiert, um die Aktivität zusammen mit der Propandiol-Oxidoreduktase-Aktivität, der Glycerin-Dehydrogenase-Aktivität und der Dihydroxyaceton-Kinase-Aktivität zu exprimieren.

4. Wirtszelle nach Anspruch 1, wobei die Wirtszelle durch Einführung zusätzlicher Gene verändert wurde, die eine Glycerin-Dehydratase kodieren, um die Glycerin-Dehydratase-Aktivität zusammen mit der Propandiol-Oxidoreduktase-Aktivität, der Glycerin-Dehydrogenase-Aktivität und der Dihydroxyaceton-Kinase-Aktivität zu exprimieren.

5. Wirtszelle nach Anspruch 1, wobei die Wirtszelle durch Einführung zusätzlicher Gene verändert wurde, die eine Aldo-Keto-Reduktase kodieren, um die Aldo-Keto-Reduktase-Aktivität zusammen mit der Propandiol-Oxidoreduktase-Aktivität, der Glycerin-Dehydrogenase-Aktivität und der Dihydroxyaceton-Kinase-Aktivität zu exprimieren.

6. Wirtszelle nach einem der vorhergehenden Ansprüche, wobei die Wirtszelle im Metabolismus mindestens eines der folgenden Stoffe einen Defekt aufweist:
i) Arabinose
ii) Methylglyoxal
iii) Dihydroxyacetonphosphat.

7. Wirtszelle nach einem der vorhergehenden Ansprüche, die große Mengen von 1,2-Propandiol, aber im Wesentlichen kein 1,3-Propandiol produziert, wenn sie auf Glycerin als einziger Kohlenstoffquelle gezüchtet wird.

8. Wirtszelle nach Anspruch 7, die E.coli ist.

9. Verfahren zur Herstellung von 1,2-Propandiol, umfassend das Züchten einer Wirtszelle nach einem der Ansprüche 1 bis 8 in einem geeigneten Wachstumsmedium, das Glycerin enthält, wonach das hergestellte 1,2-Propandiol gewonnen und gegebenenfalls aufgereinigt wird.

## Revendications

1. Cellule hôte modifiée pour produire des taux élevés de 1,2-propanediol lorsqu'elle est cultivée sur du glycérol en tant qu'unique source de carbone, dans lesquelles ladite cellule hôte a été modifiée pour introduire un gène codant pour une activité propanediol oxydoréductase (fucO), un gène codant pour une glycérol déshydrogénase (gldA) et un gène codant pour une dihydroxyacétone kinase (dhaK) et dans laquelle le gène codant pour l'activité dihydroxyacétone kinase est isolé dans une souche de Citrobacter.

2. Cellule hôte selon la revendication 1, dans laquelle le glycérol a un degré de pureté compris entre 80 % et 90 %.

3. Cellule hôte selon la revendication 1, dans laquelle ladite cellule hôte a été modifiée par introduction d'un gène supplémentaire codant pour une méthylglyoxalsynthase (mgsA) de sorte à exprimer ladite activité en même temps que l'activité propanediol oxydoréductase, l'activité glycérol deshydrogénase et l'activité dihydroxyacétone kinase.

4. Cellule hôte selon la revendication 1, dans laquelle ladite cellule hôte a été modifiée par introduction des gènes supplémentaires codant pour une glycérol déshydratase de sorte à exprimer ladite activité glycérol déshydratase en même temps que l'activité propanediol oxydoréductase, l'activité glycérol deshydrogénase et l'activité dihydroxyacétone kinase.

5. Cellule hôte selon la revendication 1, dans laquelle ladite cellule hôte a été modifiée par introduction de gènes supplémentaires codant pour une aldo-céto-réductase de sorte à exprimer ladite activité aldo-céto-réductase en même temps que l'activité propanediol oxydoréductase, l'activité glycérol deshydrogénase et l'activité dihydroxyacétone kinase.

6. Cellule hôte selon l'une quelconque des revendications précédentes, dans laquelle ladite cellule hôte est déficiente dans au moins le métabolisme des composés choisis dans le groupe constitué par :
i) l'arabinose
ii) le méthylglyoxal
iii) la dihydroxyacétone phosphate.

7. Cellule hôte selon l'une quelconque des revendications précédentes, qui produit des taux élevés de 1,2-propanediol lorsqu'elle est cultivée sur du glycérol en tant qu'unique source de carbone, mais essentiellement pas de 1,3-propanediol.

8. Cellule hôte selon la revendication 7, qui est E. coli.

9. Méthode de production de 1,2-propanediol comprenant la culture d'une cellule hôte selon l'une quelconque des revendications 1 à 8 dans un milieu de culture approprié contenant du glycérol, après quoi le 1,2-propanediol produit est récupéré et, de manière optionnelle, purifié.


### SEQUENCE LISTING

<110> Clariant International Ltd
<120> Production Method
<130> PA-3029/001 BS
<160> 34
<170> PatentIn version 3.4
<210> 1
   <211> 51
   <212> DNA
   <213> Artificial
<220>
   <223> primer gldh-for1
<400> 1
   ggggacgtca agaaggagat atacatatgg accgcattat tcaatcaccg g 51
<210> 2
   <211> 38
   <212> DNA
   <213> Artificial
<220>
   <223> primer gldh-rev1
<400> 2
   gggactattt aaattattcc cactcttgca ggaaacgc 38
<210> 3
   <211> 56
   <212> DNA
   <213> Artificial
<220>
   <223> primer dhaK-for1
<400> 3
   gggactattt aaataagaag gagatataca tatgtctcaa ttctttttta accaac 56
<210> 4
   <211> 35
   <212> DNA
   <213> Artificial
<220>
   <223> primer dhaK_rev1
<400> 4
   ggggcgcgcc ttagcccagc tcactctccg ctagc 35
<210> 5
   <211> 52
   <212> DNA
   <213> Artificial
<220>
   <223> primer fucO-for1
<400> 5
   ggggcctagg aagaaggaga tatacatatg atggctaaca gaatgattct ga 52
<210> 6
   <211> 36
   <212> DNA
   <213> Artificial
<220>
   <223> primer fucO_rev1
<400> 6
   actgcccggg cttaccaggc ggtatggtaa agctct 36
<210> 7
   <211> 53
   <212> DNA
   <213> Artificial
<220>
   <223> primer mgsA_xhol_for
<400> 7
   tgctcgagta ggcctaagaa ggagatatac atatgtacat tatggaactg acg 53
<210> 8
   <211> 28
   <212> DNA
   <213> Artificial
<220>
   <223> primer mgsA_xhol_rev
<400> 8
   atctcgagtt acttcagacg gtccgcga 28
<210> 9
   <211> 20
   <212> DNA
   <213> Artificial
<220>
   <223> primer mgsAKO_for
<400> 9
   cgccgattcc ggtaaagctg 20
<210> 10
   <211> 20
   <212> DNA
   <213> Artificial
<220>
   <223> primer mgsAKO_rev
<400> 10
   gatcctggcg cgttaccatc 20
<210> 11
   <211> 50
   <212> DNA
   <213> Artificial
<220>
   <223> primer dkgB_up
<400> 11
   ttggcgcgcc gaatttaagg aataaagata atggctatcc ctgcatttgg 50
<210> 12
   <211> 30
   <212> DNA
   <213> Artificial
<220>
   <223> primer dkgB_dw
<400> 12
   ttggcgcgcc cttaatccca ttcaggagcc 30
<210> 13
   <211> 46
   <212> DNA
   <213> Artificial
<220>
   <223> primer dkgA_up
<400> 13
   ttggcgcgcc gaatttaagg aataaagata atggctaatc caaccg 46
<210> 14
   <211> 31
   <212> DNA
   <213> Artificial
<220>
   <223> primer dkgA_dw
<400> 14
   ttggcgcgcc cttagccgcc gaactggtca g 31
<210> 15
   <211> 50
   <212> DNA
   <213> Artificial
<220>
   <223> Primer 1_aldA
<400> 15
   aacaatgtat tcaccgaaaa caaacatata aatcacagga gtcgcccatg 50
<210> 16
   <211> 50
   <212> DNA
   <213> Artificial
<220>
   <223> Primer 2_aldA
<400> 16
   gaggaaaaaa cctccgcctc tttcactcat taagactgta aataaaccac 50
<210> 17
   <211> 50
   <212> DNA
   <213> Artificial
<220>
   <223> Primer 1_ldhA
<400> 17
   ctcccctgga atgcagggga gcggcaagat taaaccagtt cgttcgggca 50
<210> 18
   <211> 50
   <212> DNA
   <213> Artificial
<220>
   <223> Primer 2_ldhA
<400> 18
   tatttttagt agcttaaatg tgattcaaca tcactggaga aagtcttatg 50
<210> 19
   <211> 50
   <212> DNA
   <213> Artificial
<220>
   <223> Primer 1_rpoS
<400> 19
   tgagactggc ctttctgaca gatgcttact tactcgcgga acagcgcttc 50
<210> 20
   <211> 50
   <212> DNA
   <213> Artificial
<220>
   <223> Primer 2_rpoS
<400> 20
   cttttgcttg aatgttccgt caagggatca cgggtaggag ccaccttatg 50
<210> 21
   <211> 50
   <212> DNA
   <213> Artificial
<220>
   <223> Primer 1_gloA
<400> 21
   tactaaaaca acattttgaa tctgttagcc attttgagga taaaaagatg 50
<210> 22
   <211> 50
   <212> DNA
   <213> Artificial
<220>
   <223> Primer 2_gloA
<400> 22
   ggcgcgatga gttcacgccc ggcaggagat tagttgccca gaccgcgacc 50
<210> 23
   <211> 50
   <212> DNA
   <213> Artificial
<220>
   <223> Primer 1_gloB
<400> 23
   cgaacggagc cgatgacaag aaagttttat cagaacctat ctttctttga 50
<210> 24
   <211> 50
   <212> DNA
   <213> Artificial
<220>
   <223> Primer 2_gloB
<400> 24
   cttgccggtt tcatcacaac cttccgtttc acactgagag gtaatctatg 50
<210> 25
   <211> 50
   <212> DNA
   <213> Artificial
<220>
   <223> Primer 1_araB
<400> 25
   aattatcaaa aatcgtcatt atcgtgtcct tatagagtcg caacggcctg 50
<210> 26
   <211> 50
   <212> DNA
   <213> Artificial
<220>
   <223> Primer 2_araB
<400> 26
   actctctact gtttctccat acccgttttt ttggatggag tgaaacgatg 50
<210> 27
   <211> 1659
   <212> DNA
   <213> Citrobacter freundii
<400> 27
   atgtctcaat tcttttttaa ccaacgcacc catcttgtga gcgacgtcat cgacggggcg      60
   attatcgcca gcccatggaa taacctggcg cgtctggaaa gcgatccggc cattcgcatc    120
   gtggtccgtc gtgaccttaa taaaaataac gtagcggtca tttccggcgg cggttcggga    180
   cacgaacccg cgcacgttgg gtttatcggt aaaggcatgc taaccgctgc ggtctgcggc    240
   gacgttttcg cctccccgag cgtggatgct gtactgaccg cgattcaggc ggtgaccggt    300
   gaggctggct gtttgttgat tgtgaaaaac tacaccggtg accgtcttaa tttcggtctc    360
   gccgccgaga aggcgcgtcg ccttggctat aacgttgaaa tgctgattgt cggcgacgac    420
   atctccctgc cggataacaa acacccacgt ggcattgcgg gaactatcct ggtgcataaa    480
   atcgcaggct attttgccga acgcggctat aacctcgcca ccgtcctgcg tgaagcgcag    540
   tacgcagcca gcaacacctt tagcctgggc gtagcgcttt ccagctgtca tctgccgcaa    600
   gaaaccgacg cagcccctcg tcatcatccg ggtcatgcgg agctgggtat gggaattcac    660
   ggcgaaccag gcgcatcggt tatcgacacc caaaacagtg cgcaagtggt aaacctgatg    720
   gtggataaac tgctggccgc cctgcctgaa accggtcgtc tggcggtgat gattaataat    780
   cttggcggcg tttccgtggc cgaaatggcc atcatcaccc gcgaactcgc cagcagcccg    840
   ctgcactcgc gtatcgactg gctaattggc ccggcctcgc tggtcaccgc gctggatatg    900
   aaaggcttct cactgacggc catcgtgctg gaagagagca tcgaaaaagc actgctcacc    960
   gaagtggaaa ccagcaactg gccgacgccg gtcccaccgc gtgaaatcac ctgcgtagtg   1020
   tcatctcagc gtagcgcccg cgtggaattc cagccttcgg caaacgccct ggtggccggg   1080
   attgtggagc tggtcaccgc aaccctttcc gatctggaga ctcatctgaa tgcgctggac   1140
   gccaaagtcg gcgatggcga taccggttcg acctttgccg ccggcgcgcg tgaaattgcc   1200
   agcctgctgc atcgccagca gctgccgctg aataaccttg ccacgctgtt cgcgctgatt   1260
   ggcgaacgtc tgaccgtggt gatgggcggt tccagcggtg tgctgatgtc aatcttcttt   1320
   accgccgccg ggcagaaact ggaacagggc gctaacgttg tcgaagcgct aaatacgggg   1380
   ctggcgcaga tgaagttcta cggcggcgca gacgaaggcg atcgcacgat gattgatgcg   1440
   ctgcaaccgg ccctgacctc gctgctcgca cagccgaaaa atctgcaggc cgcattcgac   1500
   gccgcgcaag cgggagccga acgaacctgt ttgtcgagca aagccaatgc gggtcgcgca   1560
   tcgtatctga gcagcgaaag cctgctcgga aatatggacc ccggcgcgca cgccgtagcg   1620
   atggtgttta aagcgctagc ggagagtgag ctgggctaa                          1659
<210> 28
   <211> 11172
   <212> DNA
   <213> Artificial
<220>
   <223> DNA-Sequence of plasmid pDP_mgdf
<400> 28
   tcgagtaggc ctaagaagga gatatacata tgtacattat ggaactgacg actcgcactt     60
   tacctgcgcg gaaacatatt gcgctggtgg cacacgatca ctgcaaacaa atgctgatga    120
   gctgggtgga acggcatcaa ccgttactgg aacaacacgt actgtatgca acaggcacta    180
   ccggtaactt aatttcccgc gcgaccggca tgaacgtcaa cgcgatgttg agtggcccaa    240
   tggggggtga ccagcaggtt ggcgcattga tctcagaagg gaaaattgat gtattgattt     300
   tcttctggga tccactaaat gccgtgccgc acgatcctga cgtgaaagcc ttgctgcgtc    360
   tggcgacggt atggaacatt ccggtcgcca ccaacgtggc aacggcagac ttcataatcc    420
   agtcgccgca tttcaacgac gcggtcgata ttctgatccc cgattatcag cgttatctcg    480
   cggaccgtct gaagtaactc gagtgacgtc aagaaggaga tatacatatg gaccgcatta    540
   ttcaatcacc gggtaaatac atccagggcg ctgatgtgat taatcgtctg ggcgaatacc    600
   tgaagccgct ggcagaacgc tggttagtgg tgggtgacaa atttgtttta ggttttgctc    660
   aatccactgt cgagaaaagc tttaaagatg ctggactggt agtagaaatt gcgccgtttg    720
   gcggtgaatg ttcgcaaaat gagatcgacc gtctgcgtgg catcgcggag actgcgcagt    780
   gtggcgcaat tctcggtatc ggtggcggaa aaaccctcga tactgccaaa gcactggcac    840
   atttcatggg tgttccggta gcgatcgcac cgactatcgc ctctaccgat gcaccgtgca    900
   gcgcattgtc tgttatctac accgatgagg gtgagtttga ccgctatctg ctgttgccaa    960
   ataacccgaa tatggtcatt gtcgacacca aaatcgtcgc tggcgcacct gcacgtctgt   1020
   tagcggcggg tatcggcgat gcgctggcaa cctggtttga agcgcgtgcc tgctctcgta 1080
   gcggcgcgac caccatggcg ggcggcaagt gcacccaggc tgcgctggca ctggctgaac 1140
   tgtgctacaa caccctgctg gaagaaggcg aaaaagcgat gcttgctgcc gaacagcatg 1200
   tagtgactcc ggcgctggag cgcgtgattg aagcgaacac ctatttgagc ggtgttggtt 1260
   ttgaaagtgg tggtctggct gcggcgcacg cagtgcataa cggcctgacc gctatcccgg 1320
   acgcgcatca ctattatcac ggtgaaaaag tggcattcgg tacgctgacg cagctggttc 1380
   tggaaaatgc gccggtggag gaaatcgaaa ccgtagctgc ccttagccat gcggtaggtt 1440
   tgccaataac tctcgctcaa ctggatatta aagaagatgt cccggcgaaa atgcgaattg 1500
   tggcagaagc ggcatgtgca gaaggtgaaa ccattcacaa catgcctggc ggcgcgacgc 1560
   cagatcaggt ttacgccgct ctgctggtag ccgaccagta cggtcagcgt ttcctgcaag 1620
   agtgggaata atttaaataa gaaggagata tacatatgtc tcaattcttt tttaaccaac 1680
   gcacccatct tgtgagcgac gtcatcgacg gggcgattat cgccagccca tggaataacc 1740
   tggcgcgtct ggaaagcgat ccggccattc gcatcgtggt ccgtcgtgac cttaataaaa 1800
   ataacgtagc ggtcatttcc ggcggcggtt cgggacacga acccgcgcac gttgggttta 1860
   tcggtaaagg catgctaacc gctgcggtct gcggcgacgt tttcgcctcc ccgagcgtgg 1920
   atgctgtact gaccgcgatt caggcggtga ccggtgaggc tggctgtttg ttgattgtga 1980
   aaaactacac cggtgaccgt cttaatttcg gtctcgccgc cgagaaggcg cgtcgccttg 2040
   gctataacgt tgaaatgctg attgtcggcg acgacatctc cctgccggat aacaaacacc 2100
   cacgtggcat tgcgggaact atcctggtgc ataaaatcgc aggctatttt gccgaacgcg 2160
   gctataacct cgccaccgtc ctgcgtgaag cgcagtacgc agccagcaac acctttagcc 2220
   tgggcgtagc gctttccagc tgtcatctgc cgcaagaaac cgacgcagcc cctcgtcatc 2280
   atccgggtca tgcggagctg ggtatgggaa ttcacggcga accaggcgca tcggttatcg 2340
   acacccaaaa cagtgcgcaa gtggtaaacc tgatggtgga taaactgctg gccgccctgc 2400
   ctgaaaccgg tcgtctggcg gtgatgatta ataatcttgg cggcgtttcc gtggccgaaa 2460
   tggccatcat cacccgcgaa ctcgccagca gcccgctgca ctcgcgtatc gactggctaa 2520
   ttggcccggc ctcgctggtc accgcgctgg atatgaaagg cttctcactg acggccatcg 2580
   tgctggaaga gagcatcgaa aaagcactgc tcaccgaagt ggaaaccagc aactggccga 2640
   cgccggtccc accgcgtgaa atcacctgcg tagtgtcatc tcagcgtagc gcccgcgtgg 2700
   aattccagcc ttcggcaaac gccctggtgg ccgggattgt ggagctggtc accgcaaccc 2760
   tttccgatct ggagactcat ctgaatgcgc tggacgccaa agtcggcgat ggcgataccg 2820
   gttcgacctt tgccgccggc gcgcgtgaaa ttgccagcct gctgcatcgc cagcagctgc 2880
   cgctgaataa ccttgccacg ctgttcgcgc tgattggcga acgtctgacc gtggtgatgg 2940
   gcggttccag cggtgtgctg atgtcaatct tctttaccgc cgccgggcag aaactggaac 3000
   agggcgctaa cgttgtcgaa gcgctaaata cggggctggc gcagatgaag ttctacggcg 3060
   gcgcagacga aggcgatcgc acgatgattg atgcgctgca accggccctg acctcgctgc 3120
   tcgcacagcc gaaaaatctg caggccgcat tcgacgccgc gcaagcggga gccgaacgaa 3180
   cctgtttgtc gagcaaagcc aatgcgggtc gcgcatcgta tctgagcagc gaaagcctgc 3240
   tcggaaatat ggaccccggc gcgcacgccg tagcgatggt gtttaaagcg ctagcggaga 3300
   gtgagctggg ctaaggcgcg ccttataacc taggaagaag gagatataca tatgatggct 3360
   aacagaatga ttctgaacga aacggcatgg tttggtcggg gtgctgttgg ggctttaacc 3420
   gatgaggtga aacgccgtgg ttatcagaag gcgctgatcg tcaccgataa aacgctggtg 3480
   caatgcggcg tggtggcgaa agtgaccgat aagatggatg ctgcagggct ggcatgggcg 3540
   atttacgacg gcgtagtgcc caacccaaca attactgtcg tcaaagaagg gctcggtgta 3600
   ttccagaata gcggcgcgga ttacctgatc gctattggtg gtggttctcc acaggatact 3660
   tgtaaagcga ttggcattat cagcaacaac ccggagtttg ccgatgtgcg tagcctggaa 3720
   gggctttccc cgaccaataa acccagtgta ccgattctgg caattcctac cacagcaggt 3780
   actgcggcag aagtgaccat taactacgtg atcactgacg aagagaaacg gcgcaagttt 3840
   gtttgcgttg atccgcatga tatcccgcag gtggcgttta ttgacgctga catgatggat 3900
   ggtatgcctc cagcgctgaa agctgcgacg ggtgtcgatg cgctcactca tgctattgag 3960
   gggtatatta cccgtggcgc gtgggcgcta accgatgcac tgcacattaa agcgattgaa 4020
   atcattgctg gggcgctgcg aggatcggtt gctggtgata aggatgccgg agaagaaatg 4080
   gcgctcgggc agtatgttgc gggtatgggc ttctcgaatg ttgggttagg gttggtgcat 4140
   ggtatggcgc atccactggg cgcgttttat aacactccac acggtgttgc gaacgccatc 4200
   ctgttaccgc atgtcatgcg ttataacgct gactttaccg gtgagaagta ccgcgatatc 4260
   gcgcgcgtta tgggcgtgaa agtggaaggt atgagcctgg aagaggcgcg taatgccgct 4320
   gttgaagcgg tgtttgctct caaccgtgat gtcggtattc cgccacattt gcgtgatgtt 4380
   ggtgtacgca aggaagacat tccggcactg gcgcaggcgg cactggatga tgtttgtacc 4440
   ggtggcaacc cgcgtgaagc aacgcttgag gatattgtag agctttacca taccgcctgg 4500
   taagcccggg cattaattaa caggaaacag ctatgaccat gattacggat tcactggccg 4560
   tcgttttaca acgtcgtgac tgggaaaacc ctggcgttac ccaacttaat cgccttgcag 4620
   cacatccccc tttcgccagc tggcgtaata gcgaagaggc ccgcaccgat cgcccttccc 4680
   aacagttgcg cagcctgaat ggcgaatggc gctttgcata gttattaatt aacacgtgag 4740
   gccggccgag cggccgccac cgcggtggag gggcattaat tgctagaggg tcgaaattca 4800
   aattgtgagc ggataactat ttgaattttc tgtatgaggt tttgctaaac aactttcaac 4860
   agtttcagcg gagtgagaat agaaaggaac aactaaagga attgcgaata ataatttttt 4920
   cacgttgaaa atctccaaaa aaaaaggctc caaaaggagc ctttaattgt atcggtttat 4980
   cagcttgctt tcgaggtgaa tttcgaccct ctagaggtcg aaattcaaat tgtgagcgga 5040
   taacaatttg aattttctgt atgaggtttt gctaaacaac tttcaacagt ttcagtggag 5100
   tgagaataga aaggaacaac taaaggaatt gcgaataata attttttcac gttgaaaatc 5160
   tccaaaaaaa aaggctccaa aaggagcctt taattgtatc ggtttatcag cttgctttcg 5220
   aggtgaattt tgaccctcta gcgaaaatgc aagagcaaag acgaaaacat gccacacatg 5280
   aggaataccg attctctcat taacatattc aggccagtta tctgggctta aaagcagaag 5340
   tccaacccag ataacgatca tatacatggt tctctccaga ggttcattac tgaacactcg 5400
   tccgagaata acgagtggat cccctccaat tcgccctata gtgagtcgta ttacgcgcgc 5460
   tcactggccg tcgttttaca acgtcgtgac tgggaaaacc ctggcgttac ccaacttaat 5520
   cgccttgcag cacatccccc tttcgccagc tggcgtaata gcgaagaggc ccgcaccgat 5580
   cgcccttccc aacagttgcg cagcctgaat ggcgaatgga aattgtaagc gttaatattt 5640
   tgttaaaatt cgcgttaaat ttttgttaaa tcagctcatt ttttaaccaa taggccgact 5700
   gcgatgagtg gcagggcggg gcgtaatttt tttaaggcag ttattggtgc ccttaaacgc 5760
   ctggtgctac gcctgaataa gtgataataa gcggatgaat ggcagaaatt cgaaagcaaa 5820
   ttcgacccgg tcgtcggttc agggcagggt cgttaaatag ccgcttatgt ctattgctgg 5880
   tttaccggtt tattgactac cggaagcagt gtgaccgtgt gcttctcaaa tgcctgaggc 5940
   cagtttgctc aggctctccc cgtggaggta ataattgacg atatgatcat ttattctgcc 6000
   tcccagagcc tgataaaaac ggtgaatccg ttagcgaggt gccgccggct tccattcagg 6060
   tcgaggtggc ccggctccat gcaccgcgac gcaacgcggg gaggcagaca aggtataggg 6120
   cggcgaggcg gctacagccg atagtctgga acagcgcact tacgggttgc tgcgcaaccc 6180
   aagtgctacc ggcgcggcag cgtgacccgt gtcggcggct ccaacggctc gccatcgtcc 6240
   agaaaacacg gctcatcggg catcggcagg cgctgctgcc cgcgccgttc ccattcctcc 6300
   gtttcggtca aggctggcag gtctggttcc atgcccggaa tgccgggctg gctgggcggc 6360
   tcctcgccgg ggccggtcgg tagttgctgc tcgcccggat acagggtcgg gatgcggcgc 6420
   aggtcgccat gccccaacag cgattcgtcc tggtcgtcgt gatcaaccac cacggcggca 6480
   ctgaacaccg acaggcgcaa ctggtcgcgg ggctggcccc acgccacgcg gtcattgacc 6540
   acgtaggccg acacggtgcc ggggccgttg agcttcacga cggagatcca gcgctcggcc 6600
   accaagtcct tgactgcgta ttggaccgtc cgcaaagaac gtccgatgag cttggaaagt 6660
   gtcttctggc tgaccaccac ggcgttctgg tggcccatct gcgccacgag gtgatgcagc 6720
   agcattgccg ccgtgggttt cctcgcaata agcccggccc acgcctcatg cgctttgcgt 6780
   tccgtttgca cccagtgacc gggcttgttc ttggcttgaa tgccgatttc tctggactgc 6840
   gtggccatgc ttatctccat gcggtagggt gccgcacggt tgcggcacca tgcgcaatca 6900
   gctgcaactt ttcggcagcg cgacaacaat tatgcgttgc gtaaaagtgg cagtcaatta 6960
   cagattttct ttaacctacg caatgagcta ttgcgggggg tgccgcaatg agctgttgcg 7020
   tacccccctt ttttaagttg ttgattttta agtctttcgc atttcgccct atatctagtt 7080
   ctttggtgcc caaagaaggg cacccctgcg gggttccccc acgccttcgg cgcggctccc 7140
   cctccggcaa aaagtggccc ctccggggct tgttgatcga ctgcgcggcc ttcggccttg 7200
   cccaaggtgg cgctgccccc ttggaacccc cgcactcgcc gccgtgaggc tcggggggca 7260
   ggcgggcggg cttcgccttc gactgccccc actcgcatag gcttgggtcg ttccaggcgc 7320
   gtcaaggcca agccgctgcg cggtcgctgc gcgagccttg acccgccttc cacttggtgt 7380
   ccaaccggca agcgaagcgc gcaggccgca ggccggaggc ttttccccag agaaaattaa 7440
   aaaaattgat ggggcaaggc cgcaggccgc gcagttggag ccggtgggta tgtggtcgaa 7500
   ggctgggtag ccggtgggca atccctgtgg tcaagctcgt gggcaggcgc agcctgtcca 7560
   tcagcttgtc cagcagggtt gtccacgggc cgagcgaagc gagccagccg gtggccgctc 7620
   gcggccatcg tccacatatc cacgggctgg caagggagcg cagcgaccgc gcagggcgaa 7680
   gcccggagag caagcccgta gggcgccgca gccgccgtag gcggtcacga ctttgcgaag 7740
   caaagtctag tgagtatact caagcattga gtggcccgcc ggaggcaccg ccttgcgctg 7800
   cccccgtcga gccggttgga caccaaaagg gaggggcagg catggcggca tacgcgatca 7860
   tgcgatgcaa gaagctggcg aaaatgggca acgtggcggc cagtctcaag cacgcctacc 7920
   gcgagcgcga gacgcccaac gctgacgcca gcaggacgcc agagaacgag cactgggcgg 7980
   ccagcagcac cgatgaagcg atgggccgac tgcgcgagtt gctgccagag aagcggcgca 8040
   aggacgctgt gttggcggtc gagtacgtca tgacggccag cccggaatgg tggaagtcgg 8100
   ccagccaaga acagcaggcg gcgttcttcg agaaggcgca caagtggctg gcggacaagt 8160
   acggggcgga tcgcatcgtg acggccagca tccaccgtga cgaaaccagc ccgcacatga 8220
   ccgcgttcgt ggtgccgctg acgcaggacg gcaggctgtc ggccaaggag ttcatcggca 8280
   acaaagcgca gatgacccgc gaccagacca cgtttgcggc cgctgtggcc gatctagggc 8340
   tgcaacgggg catcgagggc agcaaggcac gtcacacgcg cattcaggcg ttctacgagg 8400
   ccctggagcg gccaccagtg ggccacgtca ccatcagccc gcaagcggtc gagccacgcg 8460
   cctatgcacc gcagggattg gccgaaaagc tgggaatctc aaagcgcgtt gagacgccgg 8520
   aagccgtggc cgaccggctg acaaaagcgg ttcggcaggg gtatgagcct gccctacagg 8580
   ccgccgcagg agcgcgtgag atgcgcaaga aggccgatca agcccaagag acggcccgag 8640
   accttcggga gcgcctgaag cccgttctgg acgccctggg gccgttgaat cgggatatgc 8700
   aggccaaggc cgccgcgatc atcaaggccg tgggcgaaaa gctgctgacg gaacagcggg 8760
   aagtccagcg ccagaaacag gcccagcgcc agcaggaacg cgggcgcgca catttccccg 8820
   aaaagtgcca cctggcggcg ttgtgacaat ttaccgaaca actccgcggc cgggaagccg 8880
   atctcggctt gaacgaattg ttaggtggcg gtacttgggt cgatatcaaa gtgcatcact 8940
   tcttcccgta tgcccaactt tgtatagaga gccactgcgg gatcgtcacc gtaatctgct 9000
   tgcacgtaga tcacataagc accaagcgcg ttggcctcat gcttgaggag attgatgagc 9060
   gcggtggcaa tgccctgcct ccggtgctcg ccggagactg cgagatcata gatatagatc 9120
   tcactacgcg gctgctcaaa cctgggcaga acgtaagccg cgagagcgcc aacaaccgct 9180
   tcttggtcga aggcagcaag cgcgatgaat gtcttactac ggagcaagtt cccgaggtaa 9240
   tcggagtccg gctgatgttg ggagtaggtg gctacgtctc cgaactcacg accgaaaaga 9300
   tcaagagcag cccgcatgga tttgacttgg tcagggccga gcctacatgt gcgaatgatg 9360
   cccatacttg agccacctaa ctttgtttta gggcgactgc cctgctgcgt aacatcgttg 9420
   ctgctgcgta acatcgttgc tgctccataa catcaaacat cgacccacgg cgtaacgcgc 9480
   ttgctgcttg gatgcccgag gcatagactg tacaaaaaaa cagtcataac aagccatgaa 9540
   aaccgccact gcgccgttac caccgctgcg ttcggtcaag gttctggacc agttgcgtga 9600
   gcgcatacgc tacttgcatt acagtttacg aaccgaacag gcttatgtca actgggttcg 9660
   tgccttcatc cgtttccacg gtgtgcgtcc atgggcaaat attatacgca aggcgacaag 9720
   gtgctgatgc cgctggcgat tcaggttcat catgccgttt gtgatggctt ccatgtcggc 9780
   agaatgctta atgaattaca acagttttta tgcataatgt gcctgtcaaa tggacgaagc 9840
   agggattctg caaaccctat gctactccgt caagccgtca attgtctgat tcgttaccaa 9900
   ttatgacaac ttgacggcta catcattcac tttttcttca caaccggcac ggaactcgct 9960
   cgggctggcc ccggtgcatt ttttaaatac ccgcgagaaa tagagttgat cgtcaaaacc 10020
   aacattgcga ccgacggtgg cgataggcat ccgggtggtg ctcaaaagca gcttcgcctg 10080
   gctgatacgt tggtcctcgc gccagcttaa gacgctaatc cctaactgct ggcggaaaag 10140
   atgtgacaga cgcgacggcg acaagcaaac atgctgtgcg acgctggcga tatcaaaatt 10200
   gctgtctgcc aggtgatcgc tgatgtactg acaagcctcg cgtacccgat tatccatcgg 10260
   tggatggagc gactcgttaa tcgcttccat gcgccgcagt aacaattgct caagcagatt 10320
   tatcgccagc agctccgaat agcgcccttc cccttgcccg gcgttaatga tttgcccaaa 10380
   caggtcgctg aaatgcggct ggtgcgcttc atccgggcga aagaaccccg tattggcaaa 10440
   tattgacggc cagttaagcc attcatgcca gtaggcgcgc ggacgaaagt aaacccactg 10500
   gtgataccat tcgcgagcct ccggatgacg accgtagtga tgaatctctc ctggcgggaa 10560
   cagcaaaata tcacccggtc ggcaaacaaa ttctcgtccc tgatttttca ccaccccctg 10620
   accgcgaatg gtgagattga gaatataacc tttcattccc agcggtcggt cgataaaaaa 10680
   atcgagataa ccgttggcct caatcggcgt taaacccgcc accagatggg cattaaacga 10740
   gtatcccggc agcaggggat cattttgcgc ttcagccata cttttcatac tcccgccatt 10800
   cagagaagaa accaattgtc catattgcat cagacattgc cgtcactgcg tcttttactg 10860
   gctcttctcg ctaaccaaac cggtaacccc gcttattaaa agcattctgt aacaaagcgg 10920
   gaccaaagcc atgacaaaaa cgcgtaacaa aagtgtctat aatcacggca gaaaagtcca 10980
   cattgattat ttgcacggcg tcacactttg ctatgccata gcatttttat ccataagatt 11040
   agcggatcct acctgacgct ttttatcgca actctctact gtttctccat acccgttttt 11100
   tggtacccaa gtcgaccctg cagggtttaa accagtattc aggtagctgt tgagcctggg 11160
   gcggtagcgt gc 11172
<210> 29
   <211> 367
   <212> PRT
   <213> Escherichia coli
<400> 29
   Met Asp Arg Ile Ile Gln Ser Pro Gly Lys Tyr Ile Gln Gly Ala Asp
   1               5                   10                  15
   Val Ile Asn Arg Leu Gly Glu Tyr Leu Lys Pro Leu Ala Glu Arg Trp
               20                  25                  30
   Leu Val Val Gly Asp Lys Phe Val Leu Gly Phe Ala Gln Ser Thr Val
           35                  40                  45
   Glu Lys Ser Phe Lys Asp Ala Gly Leu Val Val Glu Ile Ala Pro Phe
       50                  55                  60
   Gly Gly Glu Cys Ser Gln Asn Glu Ile Asp Arg Leu Arg Gly Ile Ala
   65 70 75 80
   Glu Thr Ala Gln Cys Gly Ala Ile Leu Gly Ile Gly Gly Gly Lys Thr
   85 90 95
   Leu Asp Thr Ala Lys Ala Leu Ala His Phe Met Gly Val Pro Val Ala
   100 105 110
   Ile Ala Pro Thr Ile Ala Ser Thr Asp Ala Pro Cys Ser Ala Leu Ser
   115 120 125
   Val Ile Tyr Thr Asp Glu Gly Glu Phe Asp Arg Tyr Leu Leu Leu Pro
   130 135 140
   Asn Asn Pro Asn Met Val Ile Val Asp Thr Lys Ile Val Ala Gly Ala
   145 150 155 160
   Pro Ala Arg Leu Leu Ala Ala Gly Ile Gly Asp Ala Leu Ala Thr Trp
   165 170 175
   Phe Glu Ala Arg Ala Cys Ser Arg Ser Gly Ala Thr Thr Met Ala Gly
   180 185 190
   Gly Lys Cys Thr Gln Ala Ala Leu Ala Leu Ala Glu Leu Cys Tyr Asn
   195 200 205
   Thr Leu Leu Glu Glu Gly Glu Lys Ala Met Leu Ala Ala Glu Gln His
   210 215 220
   Val Val Thr Pro Ala Leu Glu Arg Val Ile Glu Ala Asn Thr Tyr Leu
   225 230 235 240
   Ser Gly Val Gly Phe Glu Ser Gly Gly Leu Ala Ala Ala His Ala Val
   245 250 255
   His Asn Gly Leu Thr Ala Ile Pro Asp Ala His His Tyr Tyr His Gly
   260 265 270
   Glu Lys Val Ala Phe Gly Thr Leu Thr Gln Leu Val Leu Glu Asn Ala
   275 280 285
   Pro Val Glu Glu Ile Glu Thr Val Ala Ala Leu Ser His Ala Val Gly
   290 295 300
   Leu Pro Ile Thr Leu Ala Gln Leu Asp Ile Lys Glu Asp Val Pro Ala
   305 310 315 320
   Lys Met Arg Ile Val Ala Glu Ala Ala Cys Ala Glu Gly Glu Thr Ile
                   325                 330                 335
   His Asn Met Pro Gly Gly Ala Thr Pro Asp Gln Val Tyr Ala Ala Leu
               340                 345                 350
   Leu Val Ala Asp Gln Tyr Gly Gln Arg Phe Leu Gln Glu Trp Glu
           355                 360                 365
<210> 30
   <211> 552
   <212> PRT
   <213> Escherichia coli
<400> 30
   Met Ser Gln Phe Phe Phe Asn Gln Arg Thr His Leu Val Ser Asp Val
   1               5                   10                  15
   Ile Asp Gly Ala Ile Ile Ala Ser Pro Trp Asn Asn Leu Ala Arg Leu
               20                  25                  30
   Glu Ser Asp Pro Ala Ile Arg Ile Val Val Arg Arg Asp Leu Asn Lys
           35                  40                  45
   Asn Asn Val Ala Val Ile Ser Gly Gly Gly Ser Gly His Glu Pro Ala
       50                  55                  60
   His Val Gly Phe Ile Gly Lys Gly Met Leu Thr Ala Ala Val Cys Gly
   65                  70                  75                  80
   Asp Val Phe Ala Ser Pro Ser Val Asp Ala Val Leu Thr Ala Ile Gln
                   85                  90                  95
   Ala Val Thr Gly Glu Ala Gly Cys Leu Leu Ile Val Lys Asn Tyr Thr
               100                 105                 110
   Gly Asp Arg Leu Asn Phe Gly Leu Ala Ala Glu Lys Ala Arg Arg Leu
           115                 120                 125
   Gly Tyr Asn Val Glu Met Leu Ile Val Gly Asp Asp Ile Ser Leu Pro
       130                 135                 140
   Asp Asn Lys His Pro Arg Gly Ile Ala Gly Thr Ile Leu Val His Lys
   145                 150                 155                 160
   Ile Ala Gly Tyr Phe Ala Glu Arg Gly Tyr Asn Leu Ala Thr Val Leu
   165 170 175
   Arg Glu Ala Gln Tyr Ala Ala Ser Asn Thr Phe Ser Leu Gly Val Ala
   180 185 190
   Leu Ser Ser Cys His Leu Pro Gln Glu Thr Asp Ala Ala Pro Arg His
   195 200 205
   His Pro Gly His Ala Glu Leu Gly Met Gly Ile His Gly Glu Pro Gly
   210 215 220
   Ala Ser Val Ile Asp Thr Gln Asn Ser Ala Gln Val Val Asn Leu Met
   225 230 235 240
   Val Asp Lys Leu Leu Ala Ala Leu Pro Glu Thr Gly Arg Leu Ala Val
   245 250 255
   Met Ile Asn Asn Leu Gly Gly Val Ser Val Ala Glu Met Ala Ile Ile
   260 265 270
   Thr Arg Glu Leu Ala Ser Ser Pro Leu His Ser Arg Ile Asp Trp Leu
   275 280 285
   Ile Gly Pro Ala Ser Leu Val Thr Ala Leu Asp Met Lys Gly Phe Ser
   290 295 300
   Leu Thr Ala Ile Val Leu Glu Glu Ser Ile Glu Lys Ala Leu Leu Thr
   305 310 315 320
   Glu Val Glu Thr Ser Asn Trp Pro Thr Pro Val Pro Pro Arg Glu Ile
   325 330 335
   Thr Cys Val Val Ser Ser Gln Arg Ser Ala Arg Val Glu Phe Gln Pro
   340 345 350
   Ser Ala Asn Ala Leu Val Ala Gly Ile Val Glu Leu Val Thr Ala Thr
   355 360 365
   Leu Ser Asp Leu Glu Thr His Leu Asn Ala Leu Asp Ala Lys Val Gly
   370 375 380
   Asp Gly Asp Thr Gly Ser Thr Phe Ala Ala Gly Ala Arg Glu Ile Ala
   385 390 395 400
   Ser Leu Leu His Arg Gln Gln Leu Pro Leu Asn Asn Leu Ala Thr Leu
   405 410 415
   Phe Ala Leu Ile Gly Glu Arg Leu Thr Val Val Met Gly Gly Ser Ser
   420 425 430
   Gly Val Leu Met Ser Ile Phe Phe Thr Ala Ala Gly Gln Lys Leu Glu
   435 440 445
   Gln Gly Ala Asn Val Val Glu Ala Leu Asn Thr Gly Leu Ala Gln Met
   450 455 460
   Lys Phe Tyr Gly Gly Ala Asp Glu Gly Asp Arg Thr Met Ile Asp Ala
   465 470 475 480
   Leu Gln Pro Ala Leu Thr Ser Leu Leu Ala Gln Pro Lys Asn Leu Gln
   485 490 495
   Ala Ala Phe Asp Ala Ala Gln Ala Gly Ala Glu Arg Thr Cys Leu Ser
   500 505 510
   Ser Lys Ala Asn Ala Gly Arg Ala Ser Tyr Leu Ser Ser Glu Ser Leu
   515 520 525
   Leu Gly Asn Met Asp Pro Gly Ala His Ala Val Ala Met Val Phe Lys
   530 535 540
   Ala Leu Ala Glu Ser Glu Leu Gly
   545 550
<210> 31
   <211> 383
   <212> PRT
   <213> Escherichia coli
<400> 31
   Met Met Ala Asn Arg Met Ile Leu Asn Glu Thr Ala Trp Phe Gly Arg
   1 5 10 15
   Gly Ala Val Gly Ala Leu Thr Asp Glu Val Lys Arg Arg Gly Tyr Gln
   20 25 30
   Lys Ala Leu Ile Val Thr Asp Lys Thr Leu Val Gln Cys Gly Val Val
   35 40 45
   Ala Lys Val Thr Asp Lys Met Asp Ala Ala Gly Leu Ala Trp Ala Ile
   50 55 60
   Tyr Asp Gly Val Val Pro Asn Pro Thr Ile Thr Val Val Lys Glu Gly
   65                  70                  75                  80
   Leu Gly Val Phe Gln Asn Ser Gly Ala Asp Tyr Leu Ile Ala Ile Gly
                   85                  90                  95
   Gly Gly Ser Pro Gln Asp Thr Cys Lys Ala Ile Gly Ile Ile Ser Asn
               100                 105                 110
   Asn Pro Glu Phe Ala Asp Val Arg Ser Leu Glu Gly Leu Ser Pro Thr
           115                 120                 125
   Asn Lys Pro Ser Val Pro Ile Leu Ala Ile Pro Thr Thr Ala Gly Thr
       130                 135                 140
   Ala Ala Glu Val Thr Ile Asn Tyr Val Ile Thr Asp Glu Glu Lys Arg
   145                 150                 155                 160
   Arg Lys Phe Val Cys Val Asp Pro His Asp Ile Pro Gln Val Ala Phe
                   165                 170                 175
   Ile Asp Ala Asp Met Met Asp Gly Met Pro Pro Ala Leu Lys Ala Ala
               180                 185                 190
   Thr Gly Val Asp Ala Leu Thr His Ala Ile Glu Gly Tyr Ile Thr Arg
           195                 200                 205
   Gly Ala Trp Ala Leu Thr Asp Ala Leu His Ile Lys Ala Ile Glu Ile
       210                 215                 220
   Ile Ala Gly Ala Leu Arg Gly Ser Val Ala Gly Asp Lys Asp Ala Gly
   225                 230                 235                 240
   Glu Glu Met Ala Leu Gly Gln Tyr Val Ala Gly Met Gly Phe Ser Asn
                   245                 250                 255
   Val Gly Leu Gly Leu Val His Gly Met Ala His Pro Leu Gly Ala Phe
               260                 265                 270
   Tyr Asn Thr Pro His Gly Val Ala Asn Ala Ile Leu Leu Pro His Val
           275                 280                 285
   Met Arg Tyr Asn Ala Asp Phe Thr Gly Glu Lys Tyr Arg Asp Ile Ala
       290                 295                 300
   Arg Val Met Gly Val Lys Val Glu Gly Met Ser Leu Glu Glu Ala Arg
   305                 310                 315                 320
   Asn Ala Ala Val Glu Ala Val Phe Ala Leu Asn Arg Asp Val Gly Ile
                   325                 330                 335
   Pro Pro His Leu Arg Asp Val Gly Val Arg Lys Glu Asp Ile Pro Ala
               340                 345                 350
   Leu Ala Gln Ala Ala Leu Asp Asp Val Cys Thr Gly Gly Asn Pro Arg
           355                 360                 365
   Glu Ala Thr Leu Glu Asp Ile Val Glu Leu Tyr His Thr Ala Trp
       370                 375                 380
<210> 32
   <211> 152
   <212> PRT
   <213> Escherichia coli
<400> 32
<210> 33
   <211> 275
   <212> PRT
   <213> Escherichia coli
<400> 33
   Met Ala Asn Pro Thr Val Ile Lys Leu Gln Asp Gly Asn Val Met Pro
   1               5                   10                  15
   Gln Leu Gly Leu Gly Val Trp Gln Ala Ser Asn Glu Glu Val Ile Thr
               20                  25                  30
   Ala Ile Gln Lys Ala Leu Glu Val Gly Tyr Arg Ser Ile Asp Thr Ala
           35                  40                  45
   Ala Ala Tyr Lys Asn Glu Glu Gly Val Gly Lys Ala Leu Lys Asn Ala
       50                  55                  60
   Ser Val Asn Arg Glu Glu Leu Phe Ile Thr Thr Lys Leu Trp Asn Asp
   65                  70                  75                  80
   Asp His Lys Arg Pro Arg Glu Ala Leu Leu Asp Ser Leu Lys Lys Leu
                   85                  90                  95
   Gln Leu Asp Tyr Ile Asp Leu Tyr Leu Met His Trp Pro Val Pro Ala
               100                 105                 110
   Ile Asp His Tyr Val Glu Ala Trp Lys Gly Met Ile Glu Leu Gln Lys
           115                 120                 125
   Glu Gly Leu Ile Lys Ser Ile Gly Val Cys Asn Phe Gln Ile His His
       130                 135                 140
   Leu Gln Arg Leu Ile Asp Glu Thr Gly Val Thr Pro Val Ile Asn Gln
   145                 150                 155                 160
   Ile Glu Leu His Pro Leu Met Gln Gln Arg Gln Leu His Ala Trp Asn
                   165                 170                 175
   Ala Thr His Lys Ile Gln Thr Glu Ser Trp Ser Pro Leu Ala Gln Gly
               180                 185                 190
   Gly Lys Gly Val Phe Asp Gln Lys Val Ile Arg Asp Leu Ala Asp Lys
           195                 200                 205
   Tyr Gly Lys Thr Pro Ala Gln Ile Val Ile Arg Trp His Leu Asp Ser
       210                 215                 220
   Gly Leu Val Val Ile Pro Lys Ser Val Thr Pro Ser Arg Ile Ala Glu
   225                 230                 235                 240
   Asn Phe Asp Val Trp Asp Phe Arg Leu Asp Lys Asp Glu Leu Gly Glu
                   245                 250                 255
   Ile Ala Lys Leu Asp Gln Gly Lys Arg Leu Gly Pro Asp Pro Asp Gln
               260                 265                 270
   Phe Gly Gly
           275
<210> 34
   <211> 267
   <212> PRT
   <213> Escherichia coli
<400> 34
   Met Ala Ile Pro Ala Phe Gly Leu Gly Thr Phe Arg Leu Lys Asp Asp
   1               5                   10                  15
   Val Val Ile Ser Ser Val Ile Thr Ala Leu Glu Leu Gly Tyr Arg Ala
               20                  25                  30
   Ile Asp Thr Ala Gln Ile Tyr Asp Asn Glu Ala Ala Val Gly Gln Ala
           35                  40                  45
   Ile Ala Glu Ser Gly Val Pro Arg His Glu Leu Tyr Ile Thr Thr Lys
       50                  55                  60
   Ile Trp Ile Glu Asn Leu Ser Lys Asp Lys Leu Ile Pro Ser Leu Lys
   65                  70                  75                  80
   Glu Ser Leu Gln Lys Leu Arg Thr Asp Tyr Val Asp Leu Thr Leu Ile
                   85                  90                  95
   His Trp Pro Ser Pro Asn Asp Glu Val Ser Val Glu Glu Phe Met Gln
               100                 105                 110
   Ala Leu Leu Glu Ala Lys Lys Gln Gly Leu Thr Arg Glu Ile Gly Ile
           115                 120                 125
   Ser Asn Phe Thr Ile Pro Leu Met Glu Lys Ala Ile Ala Ala Val Gly
       130                 135                 140
   Ala Glu Asn Ile Ala Thr Asn Gln Ile Glu Leu Ser Pro Tyr Leu Gln
   145                 150                 155                 160
   Asn Arg Lys Val Val Ala Trp Ala Lys Gln His Gly Ile His Ile Thr
                   165                 170                 175
   Ser Tyr Met Thr Leu Ala Tyr Gly Lys Ala Leu Lys Asp Glu Val Ile
               180                 185                 190
   Ala Arg Ile Ala Ala Lys His Asn Ala Thr Pro Ala Gln Val Ile Leu
           195                 200                 205
   Ala Trp Ala Met Gly Glu Gly Tyr Ser Val Ile Pro Ser Ser Thr Lys
       210                 215                 220
   Arg Lys Asn Leu Glu Ser Asn Leu Lys Ala Gln Asn Leu Gln Leu Asp
   225                 230                 235                 240
   Ala Glu Asp Lys Lys Ala Ile Ala Ala Leu Asp Cys Asn Asp Arg Leu
                   245                 250                 255
   Val Ser Pro Glu Gly Leu Ala Pro Glu Trp Asp
               260                 265